(19) Europäisches Patentamt
European Patent Office
Office européen des brevets

(11) EP 1 297 835 B1

# (12) FASCICULE DE BREVET EUROPEEN

(45) Date de publication et mention de la délivrance du brevet:
**19.09.2007 Bulletin 2007/38**

(51) Int Cl.:
***A61K 31/436*** *(2006.01)* ***A61P 35/00*** *(2006.01)*
***C07D 491/04*** *(2006.01)*

(21) Numéro de dépôt: **02291862.7**

(22) Date de dépôt: **24.07.2002**

(54) **Dérivés de benzo[b]pyrano[3,2-h]acridin-7-one, leur procédé de préparation et les compositions pharmaceutiques qui les contiennent**

Benzo[b]pyrano[3,2-h]acridin-7-on Derivative, Verfahren zu ihrer Herstellung und sie enthaltende pharmazeutische Zusammensetzungen

Benzo[b]pyrano[3,2-h]acridin-7-one derivatives, processes for their preparation and pharmaceutical compositions thereof

(84) Etats contractants désignés:
**AT BE BG CH CY CZ DE DK EE ES FI FR GB GR IE IT LI LU MC NL PT SE SK TR**
Etats d'extension désignés:
**AL LT LV MK RO SI**

(30) Priorité: **25.07.2001 FR 0109910**

(43) Date de publication de la demande:
**02.04.2003 Bulletin 2003/14**

(73) Titulaire: **Les Laboratoires Servier**
**92415 Courbevoie Cédex (FR)**

(72) Inventeurs:
- **Koch, Michel**
  **78170 La Celle Saint Cloud (FR)**
- **Tillequin, Francois**
  **75014 Paris (FR)**
- **Michel, Sylvie**
  **75002 Paris (FR)**
- **Seguin, Elisabeth**
  **75014 Paris (FR)**
- **El Omri, Abdelhakim**
  **Appartement 73**
  **76000 Rouen (FR)**
- **Hickman, John**
  **75017 Paris (FR)**
- **Pierre, Alain**
  **78580 Les Alluets le Roi (FR)**
- **Pfeiffer, Bruno**
  **95320 Saint Leu la Foret (FR)**
- **Renard, Pierre**
  **78150 Le Chesnay (FR)**

(56) Documents cités:
**EP-A- 1 061 081** **WO-A-99/32491**

- **COSTES, NDINE ET AL.: "Synthesis and Cytotoxic and Antitumor Activity of Benzo(b) pyrano(3,2-h)acridin-7-one Analogues of Acronycine" J. MED CHEM., vol. 43, 24 mai 2000 (2000-05-24), pages 2395-2402, XP002198878**
- **LEONCE, STEPHANE ET AL.: "Induction od Cyclin E and Inhibition of DNA Synthesis by the novel acronycine derivative S23906-1 precede the irrevesible arrest of tumor cells in S phase leading to apoptosis" MOLECULAR PHARMACOLOGY, vol. 60, no. 6, 2001, XP000107681**
- **ELOMRI, ABDELHAKIM ET AL.: "Synthesis and Cytotoxic and Antitumor Activity of Esters in the 1,2-Dihydroxy-1,2-dihydroacroycine Series" J. MED. CHEM., vol. 39, 1996, pages 4762-4766, XP002076704**

EP 1 297 835 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne de nouveaux dérivés de benzo[*b*]pyrano[3,2-*h*]acridin-7-one, leur procédé de préparation et les compositions pharmaceutiques qui les contiennent.

Les composés de l'invention constituent des dérivés de l'acronycine qui est un alcaloïde présentant des propriétés antitumorales mises en évidence dans des modèles expérimentaux (J. Pharm.. Sci., 1966, 55 (8), 758-768). Cependant, malgré un spectre d'activité assez large, l'acronycine est peu puissant et modérément actif. De plus, ce produit présente une faible solubilité limitant sa biodisponibilité ainsi que son utilisation dans des compositions pharmaceutiques administrables par voie intraveineuse.

Diverses modifications ont été réalisées sur cette molécule, comme celles décrites dans J. Med. Chem., 1996, 39, 4762-4766 ou EP 1 042 326 et qui ont permis d'améliorer significativement la puissance, l'efficacité antitumorale et la solubilité de ces produits. Néanmoins, les besoins de la thérapeutique anticancéreuse exigent le développement constant de nouveaux agents antitumoraux, dans le but d'obtenir des médicaments à la fois plus actifs et mieux tolérés. Plus particulièrement, les tumeurs solides posent un problème majeur à la chimiothérapie anticancéreuse, de par leur résistance intrinsèque et/ou acquise, aux produits existants. De plus certain composés qui se révèlent très actifs sur certaines lignées cellulaires vont être inactifs voire toxiques sur d'autre lignées cellulaires. Il est donc primordiale d'avoir accès à une gamme la plus large possible de produits exprimant une activité cytotoxique forte afin de pouvoir disposer de traitements des plus efficaces sur l'ensemble des affections tumorales, avec des effets secondaires restreints et une action dans le temps la plus longue souhaitable.

Les composés de l'invention, outre le fait qu'ils soient nouveaux, présentent une activité cytotoxique *in vitro* et *in vivo* surprenante et supérieure à celle observée jusqu'ici. Ainsi, les composés découverts par la Demanderesse possèdent des propriétés antitumorales qui les rendent particulièrement utiles pour le traitement des cancers et notamment des tumeurs solides.

Plus particulièrement, la présente invention concerne les composés de formule (I) :

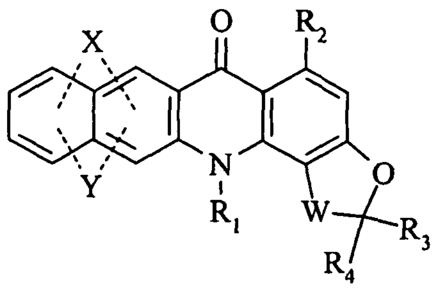

**(I)**

dans laquelle :

- **X** et **Y**, identiques ou différents, indépendamment l'un de l'autre, représentent chacun un groupement choisi parmi :
  - atome d'hydrogène, d'halogène,
  - groupement mercapto, cyano, nitro, alkyle ($C_1$-$C_6$) linéaire ou ramifié, trihalogénoalkyle ($C_1$-$C_6$) linéaire ou ramifié, trihalogénoalkylcarbonylamino ($C_1$-$C_6$) linéaire ou ramifié,
  - groupement de formules ORa, NRaRb, -NRa-C(O)-$T_1$, -O-C(O)-$T_1$, -O-$T_2$-NRaRb, -O-$T_2$-ORa, -NRa-$T_2$-NRaRb, -NRa-$T_2$-ORa, et -NRa-$T_2$-$CO_2$Ra dans lesquels :

    * **Ra** représente un groupement choisi parmi atome d'hydrogène, groupement alkyle ($C_1$-$C_6$) linéaire ou ramifié, aryle, et arylalkyle ($C_1$-$C_6$) linéaire ou ramifié,
    * **Rb** représente un groupement choisi parmi atome d'hydrogène, groupement alkyle ($C_1$-$C_6$) linéaire ou ramifié, aryle, et arylalkyle ($C_1$-$C_6$) linéaire ou ramifié,
    ou
    **Ra+Rb** forment ensemble, avec l'atome d'azote qui les portent, un hétérocycle de 5 à 6 chaînons, monocyclique, contenant éventuellement au sein du système cyclique un second hétéroatome choisi parmi oxygène et azote,
    * **$T_1$** représente un groupement choisi parmi alkyle ($C_1$-$C_6$) linéaire ou ramifié, alkènyle ($C_2$-$C_6$) linéaire ou ramifié, aryle, arylalkyle ($C_1$-$C_6$) linéaire ou ramifié, et une chaîne alkylène ($C_1$-$C_6$) linéaire ou ramifié substituée par un groupement choisi parmi -ORa et -NRaRb dans lesquels Ra et Rb sont tels que définis précédemment,

* **$T_2$** représente une chaîne alkylène ($C_1$-$C_6$) linéaire ou ramifiée,

- ou X et Y, lorsqu'ils sont en positions adjacentes, forment ensemble un groupement méthylènedioxy ou un groupement éthylènedioxy,

étant entendu que les substituants X et Y peuvent être présents sur l'un ou l'autre des deux cycles benzéniques adjacents,

- **$R_1$** représente un atome d'hydrogène ou un groupement alkyle ($C_1$-$C_6$) linéaire ou ramifié,

- **$R_2$** représente un groupement choisi parmi atome d'hydrogène, groupement alkyle ($C_1$-$C_6$) linéaire ou ramifié, ORa, NRaRb, -NRa-C(O)-$T_1$, -O-C(O)-$T_1$, -O-$T_2$-NRaRb, -O-$T_2$-ORa, -NRa-$T_2$-NRaRb, -NRa-$T_2$-ORa, et -NRa-$T_2$-$CO_2$Ra dans lesquels Ra, Rb, $T_1$ et $T_2$ sont tels que définis précédemment,

- **$R_3$, $R_4$,** identiques ou différents, indépendamment l'un de l'autre, représentent un atome d'hydrogène ou un groupement alkyle ($C_1$-$C_6$) linéaire ou ramifié,

- **W** représente un groupement de formule -CH($R_5$)-CH($R_6$)-, dans laquelle :

* **$R_5$ et/ou $R_6$,** représente(nt) un groupement choisi parmi -$W_1$-C($W_2$)-$W_3$-$T_1$, -$W_1$-S(O)$_n$-$W_3$-$T_1$ et -$W_1$-S(O)$_n$-$T_1$ dans lesquels :

- $W_1$ représente un atome d'oxygène, de soufre ou un atome d'azote substitué par un atome d'hydrogène, un groupement alkyle ($C_1$-$C_6$) linéaire ou ramifié, aryle ou arylalkyle ($C_1$-$C_6$) linéaire ou ramifié,
- $W_2$ représente un atome d'oxygène ou un atome de soufre,
- $W_3$ représente un atome d'oxygène, de soufre ou un atome d'azote substitué par un atome d'hydrogène, un groupement alkyle ($C_1$-$C_6$) linéaire ou ramifié, aryle ou arylalkyle ($C_1$-$C_6$) linéaire ou ramifié, une liaison lorsque $T_1$ représente un groupement alkényle $C_4$,
- $T_1$ est tel que défini précédemment,
- n représente un entier choisi parmi 1 ou 2,

et dans le cas où un seul des deux groupements $R_5$ et $R_6$ représente un groupement tel que défini précédemment, alors l'autre desdits groupements $R_5$ ou $R_6$ représente un groupement choisi parmi atome d'hydrogène, hydroxy, alkoxy ($C_1$-$C_6$) linéaire ou ramifié, alkylcarbonyloxy ($C_1$-$C_6$) linéaire ou ramifié, arylcarbonyloxy, arylalkylcarbonyloxy ($C_1$-$C_6$) linéaire ou ramifié, et amino éventuellement substitué par un ou deux groupements alkyle ($C_1$-$C_6$) linéaire ou ramifié, identiques ou différents, leurs énantiomères, diastéréoisomères, N-oxydes, ainsi que leurs sels d'addition à un acide ou à une base pharmaceutiquement acceptable.

Par aryle, on comprend un groupement phényle ou naphtyle, comportant éventuellement une ou plusieurs substitutions, identiques ou différentes, choisies parmi hydroxy, halogène, carboxy, nitro, amino, alkylamino ou dialkylamino ($C_1$-$C_6$) linéaire ou ramifié, alkoxy ($C_1$-$C_6$) linéaire ou ramifié, acyle ($C_1$-$C_6$) linéaire ou ramifié, et alkylcarbonyloxy ($C_1$-$C_6$) linéaire ou ramifié.

Par isomère on comprend les isomères optiques soit les énantiomères et les diastéréoisomères.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléique, citrique, ascorbique, oxalique, méthane sulfonique, camphorique, la lysine, etc...

Parmi les bases pharmaceutiquement acceptables, on peut citer à titre non limitatif l'hydroxyde de sodium, l'hydroxyde de potassium, la triéthylamine, la tertbutylamine, etc...

Selon une variante avantageuse de l'invention, les composés préférés sont les composés de formule (IA) :

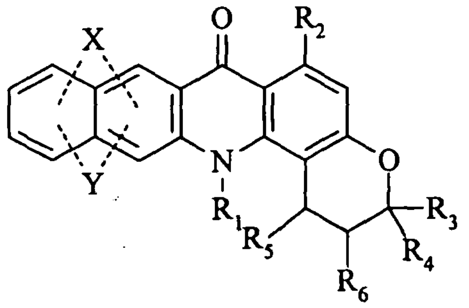

(IA)

dans laquelle X, Y, $R_1$, $R_2$, $R_3$, $R_4$, $R_5$ et $R_6$ sont tels que définis dans la formule (I).

Les composés préférés de formule (IA) sont les composés dans lesquels $R_5$ et $R_6$, identiques, représentent un groupement de formule $-W_1-C(W_2)-W_3-T_1$ ou $-W_1-S(O)_n-T_1$ dans laquelle $W_1$, $W_2$, $W_3$, $T_1$ et n sont tels que définis dans la formule (I).

D'une façon particulièrement intéressante, les composés préférés de formule (IA) sont les composés dans lesquels $R_5$ et $R_6$, identiques, représentent un groupement de formule $-W_1-C(W_2)-W_3-T_1$ dans laquelle $W_1$ représente un atome d'oxygène, $W_2$ représente un atome d'oxygène, $W_3$ représente un atome d'azote substitué par un atome d'hydrogène, un groupement alkyle ($C_1$-$C_6$) linéaire ou ramifié, aryle ou arylalkyle ($C_1$-$C_6$) linéaire ou ramifié et $T_1$ est tel que défini dans la formule (I).

D'une autre façon particulièrement intéressante, les composés préférés de formule (IA) sont les composés dans lesquels $R_5$ et $R_6$, identiques, représentent un groupement de formule $-W_1-S(O)_n-T_1$ dans laquelle $W_1$ représente un atome d'oxygène, $T_1$ est tel que défini dans la formule (I) et n est égal à 2.

Les substituants $R_3$ et $R_4$ préférés selon l'invention sont le groupement alkyle ($C_1$-$C_6$) linéaire ou ramifié.

Les substituants $R_2$ préférés selon l'invention sont les groupements choisis parmi alkoxy ($C_1$-$C_6$) linéaire ou ramifié, NRaRb, $-O-T_2-NRaRb$, $-O-T_2-ORa$, $-NRa-T_2-NRaRb$ et $-NRa-T_2-ORa$ dans lesquels Ra, Rb, $T_1$ et $T_2$ sont tels que définis dans la formule (I).

D'une façon particulièrement avantageuse les composés préférés de l'invention sont le :

- (1*S*,2*S*)-1-{[(diméthylamino)carbonyl]oxy}-6-méthoxy-3,3,14-triméthyl-7-oxo-2,3,7,14-tétrahydro-1*H*-benzo[*b*]pyrano[3,2-*h*]acridin-2-yl diméthylcarbamate
- (1*S*,2*S*)-6-méthoxy-3,3,14-triméthyl-2-{[(4-méthylphényl)sulfonyl]oxy}-7-oxo-2,3,7,14-tétrahydro-1*H*-benzo[*b*]pyrano[3,2-*h*]acridin-1-yl 4-méthylbenzènesulfonate,

Les énantiomères, diastéréoisomères, N-oxydes, ainsi que les sels d'addition à un acide ou à une base, pharmaceutiquement acceptable, des composés préférés font partie intégrante de l'invention.

La présente invention s'étend également au procédé de préparation des composés de formule (I), caractérisé en ce qu'on utilise comme produit de départ un composé de formule (VI) :

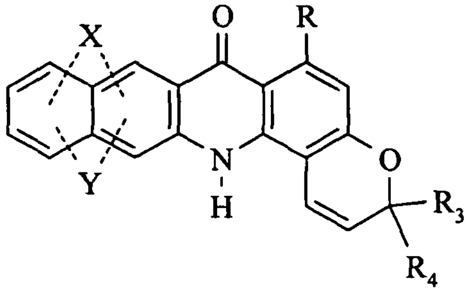

(VI)

dans laquelle X, Y, R, $R_3$ et $R_4$ sont tels que définis précédemment,
composé de formule (VI) dont l'atome d'azote est substitué ou non, par action d'un halogénure d'alkyle ou d'un sulfate de dialkyle en présence d'un agent de déprotonation, en solvant polaire aprotique ou en condition de transfert de phase, permettant d'obtenir les composés de formule (IX) :

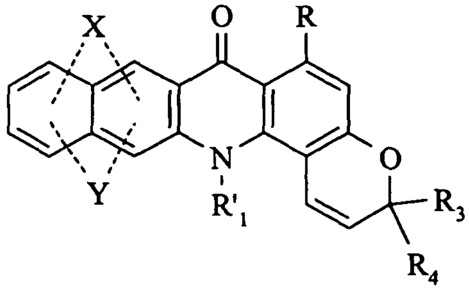

(IX)

dans laquelle X, Y, R, $R_3$ et $R_4$ sont tels que définis précédemment, et $R'_1$, représente un groupement alkyle ($C_1$-$C_6$) linéaire ou ramifié,
composés de formule (IX) qui sont soumis à l'action d'un agent alkylant ou d'un agent acylant selon des conditions classiques de la synthèse organique, pour conduire aux composés de formule (X) :

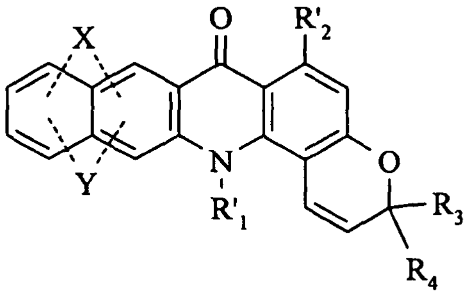

(X)

dans laquelle X, Y, $R'_1$, $R_3$ et $R_4$ sont tels que définis précédemment et $R'_2$ représente un groupement choisi parmi ORa, -O-C(O)-$T_1$, -O-$T_2$-NRaRb, -O-$T_2$-ORa dans lesquels Ra, Rb, $T_1$ et $T_2$ sont tels que définis dans la formule (I),
composés de formule (X) qui sont traités, dans le cas où $R'_2$ représente un groupement alkoxy par un composé de formule (XI):

$HNRaR_{10}$ **(XI)**

dans laquelle Ra représente un atome d'hydrogène, un groupement alkyle ($C_1$-$C_6$) linéaire ou ramifié, aryle, ou arylalkyle ($C_1$-$C_6$) linéaire ou ramifié, et $R_{10}$ représente un groupement choisi parmi Rb, -C(O)-$T_1$, -$T_2$-NRaRb, -$T_2$-ORa, et -$T_2$-$CO_2$Ra dans lesquels Ra, Rb, $T_1$ et $T_2$ sont tels que définis dans la formule (I),
pour conduire aux composés de formule (XII) :

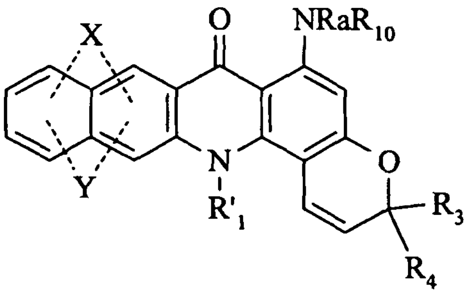

(XII)

dans laquelle X, Y, $R'_1$, $R_3$, $R_4$, Ra et $R_{10}$ sont tels que définis précédemment,
l'ensemble des composés de formule (VI), (IX), (X) et (XII) formant les composés de formule (XIII) :

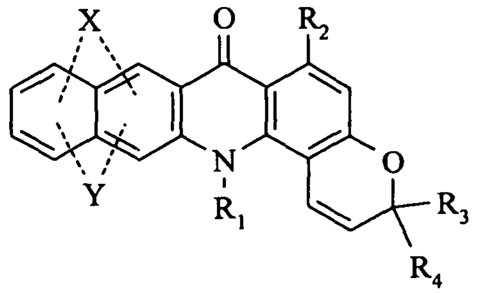

**(XIII)**

dans laquelle X, Y, $R_1$, $R_2$, $R_3$ et $R_4$ ont la même signification que dans la définition générale de la formule (I), ***composés de formule (XIII) qui sont soumis :***

a) ⇒ soit à l'action d'un composé de formule (XIV) :

Hal-C($W_2$)-$T_1$ **(XIV)**

dans laquelle Hal représente un atome d'halogène, $W_2$ et $T_1$ ont la même signification que dans la définition générale de la formule (I), pour conduire aux composés de formule (I/a), cas particulier des composés de formule (I) :

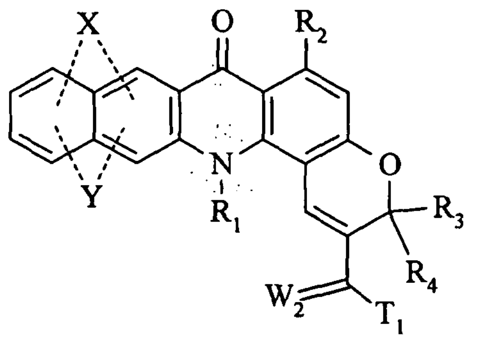

**(I/a)**

dans laquelle X, Y, $R_1$, $R_2$, $R_3$, $R_4$, $W_2$ et $T_1$ ont la même signification que précédemment,

b) ⇒ soit à l'action d'un agent oxydant des fonctions alcènes, pour conduire aux composés de formule (XV) :

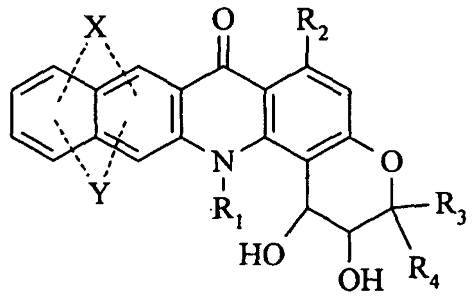

**(XV)**

dans laquelle X, Y, $R_1$, $R_2$, $R_3$ et $R_4$ sont tels que définis précédemment, composés de formule (XV) qui sont traités par un composé de formule (XVI) :

**Hal-$G_1$** **(XVI)**

dans laquelle Hal représente un atome d'halogène et $G_1$ représente un groupement choisi parmi -C($W_2$)-$W_3$-$T_1$, -C($W_2$)-$T_1$, -S(O)$_n$-$W_3$-$T_1$ et -S(O)$_n$-$T_1$ dans lesquels $W_2$, $W_3$, $T_1$ et n sont tels que définis dans la formule (I),

pour conduire aux composés de formules (I/b), (I/c) et (I/d), cas particulier des composés de formule (I) :

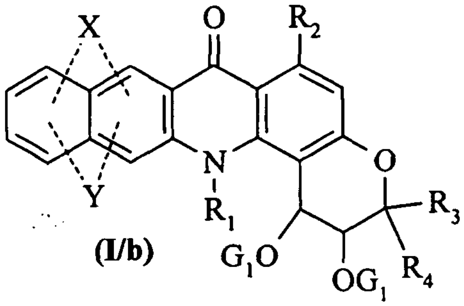

(I/b)

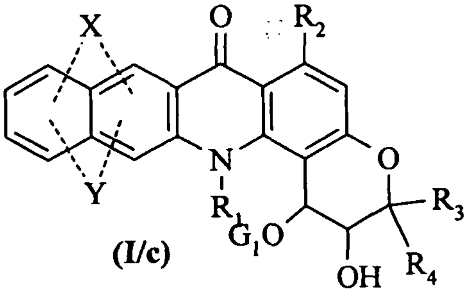

(I/c)

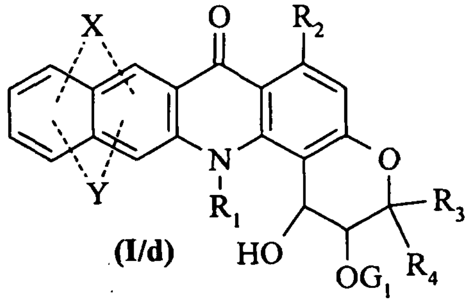

(I/d)

dans laquelle X, Y, $R_1$, $R_2$, $R_3$, $R_4$ et $G_1$ sont tels que définis précédemment, composés de formule (I/c) et/ou (I/d) qui sont soumis :

❖ soit à l'action d'un alcool de formule $R_{20}$-OH dans laquelle $R_{20}$ représente un groupement alkyle ($C_1$-$C_6$) linéaire ou ramifié, pour conduire respectivement aux composés de formule (I/c1) et (1/d1):

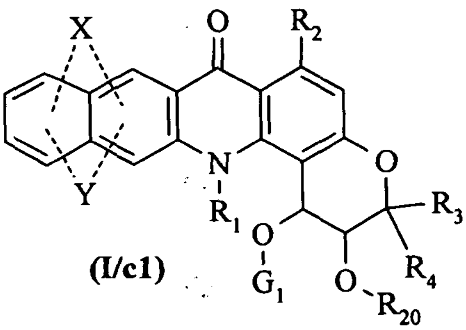

(I/c1)

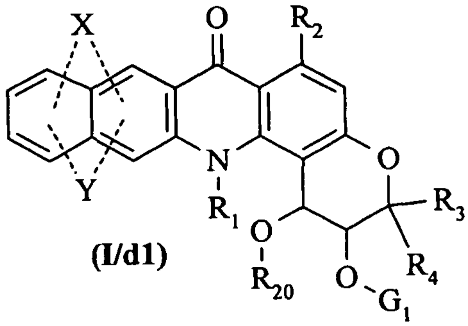

(I/d1)

dans laquelle X, Y, $R_1$, $R_2$, $R_3$, $R_4$, $G_1$ et $R_{20}$ sont tels que définis précédemment,

❖ soit à l'action d'un anhydride de formule $(R_{30}CO)_20$ dans laquelle $R_{30}$ représente un groupement alkyle ($C_1$-$C_6$) linéaire ou ramifié, aryle ou arylalkyle ($C_1$-$C_6$) linéaire ou ramifié, pour conduire respectivement aux composés de formule (I/c2) et (I/d2) :

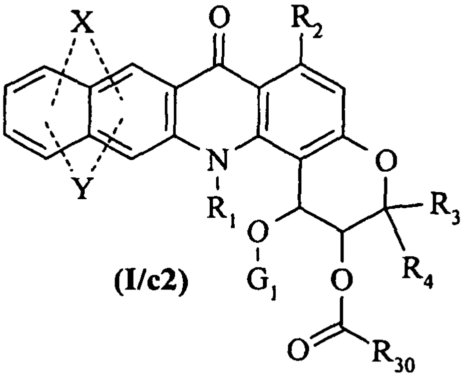

(I/c2)

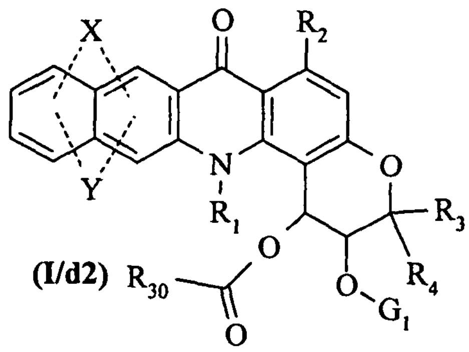

(I/d2)

dans laquelle X, Y, $R_1$, $R_2$, $R_3$, $R_4$, $G_1$ et $R_{30}$ sont tels que définis précédemment,

❖ soit à des conditions de déshydratation en milieu acide, pour conduire respectivement aux composés de formule (I/c3) et (I/d3), cas particulier des composés de formule (I) :

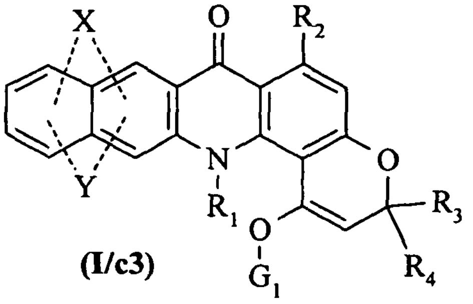

(I/c3)

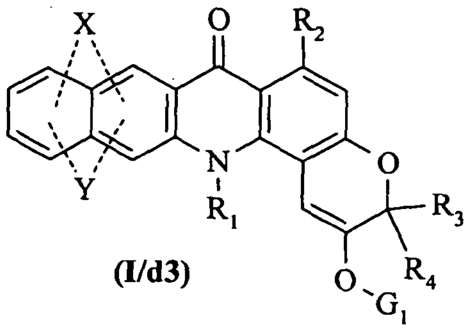

(I/d3)

dans laquelle X, Y, $R_1$, $R_2$, $R_3$, $R_4$ et $G_1$ sont tels que définis précédemment,

c) ⇒ soit à l'action d'un peracide ou du diméthyle dioxirane, pour conduire aux composés de formule (XVII) :

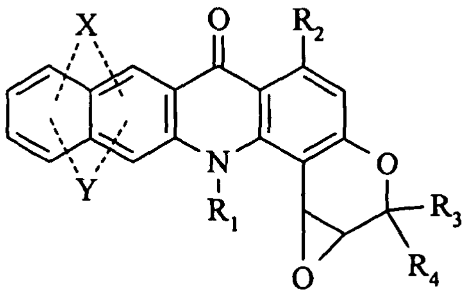

(XVII)

dans laquelle X, Y, $R_1$, $R_2$, $R_3$ et $R_4$ sont tels que définis précédemment,
composés de formule (XVII) qui sont traités par de l'ammoniac ou une amine primaire pour conduire aux composés de formule (XVIII/a) et/ou (XVIII/b) :

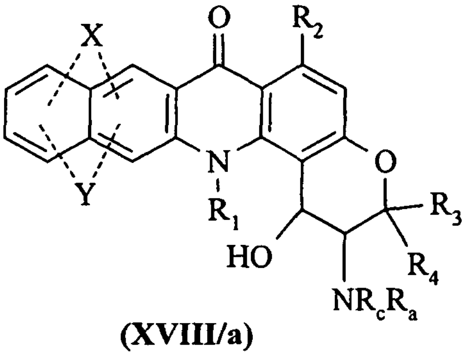

(XVIII/a)

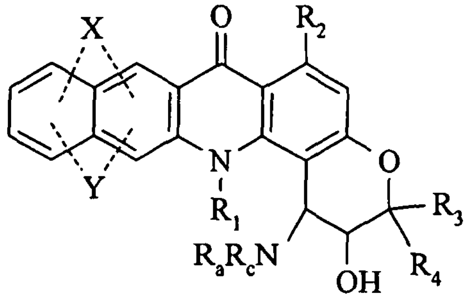

(XVIII/b)

dans lesquelles X, Y, $R_1$, $R_2$, $R_3$ et $R_4$, sont tels que définis précédemment, $R_c$ représente un atome d'hydrogène et $R_a$ représente un atome d'hydrogène dans le cas où le réactif utilisé est de l'ammoniac, ou un groupement choisi parmi alkyle ($C_1$-$C_6$) linéaire ou ramifié, aryle et arylalkyle ($C_1$-$C_6$) linéaire ou ramifié dans le cas où le réactif utilisé est une amine primaire,

composés de formules (XVIII/a) et (XVIII/b) dont la fonction alcool est protégée par un groupement protecteur des groupes hydroxy puis qui sont soumis à l'action d'un composé de formule (XVI) Hal-$G_1$ tel que défini précédemment, pour conduire respectivement aux composés de formules (XIX/a) et (XIX/b),

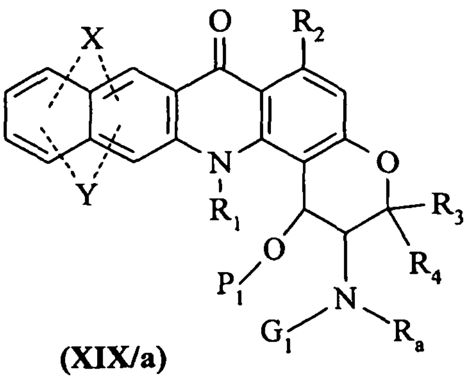

(XIX/a)

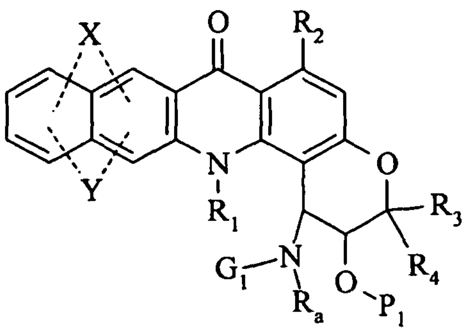

(XIX/b)

dans lesquelles X, Y, $R_1$, $R_2$, $R_3$, $R_4$, $R_a$, $G_1$ sont tels que définis précédemment, et $P_1$ représente un groupement protecteur des fonctions hydroxy,

composés de formules (XIX/a) et (XIX/b), dont on déprotège le groupement hydroxy pour conduire respectivement aux composés de formules (I/e1) et (I/f1), cas particuliers des composés de formule (I) :

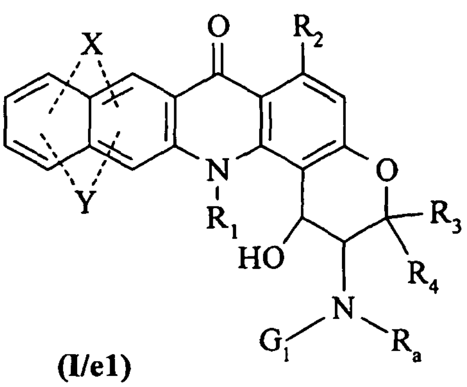

(I/e1)

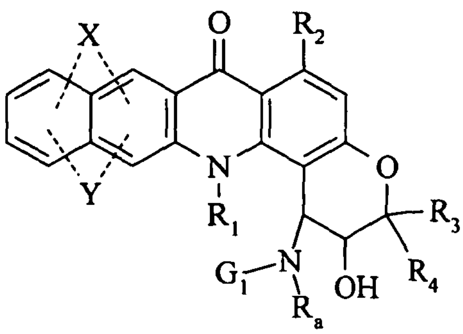

(I/f1)

dans lesquelles X, Y, $R_1$, $R_2$, $R_3$, $R_4$, $R_a$ et $G_1$ sont tels que définis précédemment,

composés de formules (I/e1) et (I/f1) qui sont soumis :

❖ soit à l'action d'un anhydride de formule $(R_{30}CO)_2O$ dans laquelle $R_{30}$ représente un groupement alkyle ($C_1$-$C_6$) linéaire ou ramifié, aryle ou arylalkyle ($C_1$-$C_6$) linéaire ou ramifié, pour conduire respectivement aux composés de formule (I/e2) et (I/f2), cas particuliers des composés de formule (I) :

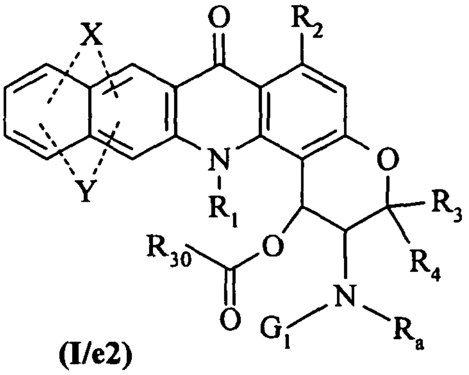

(I/e2)

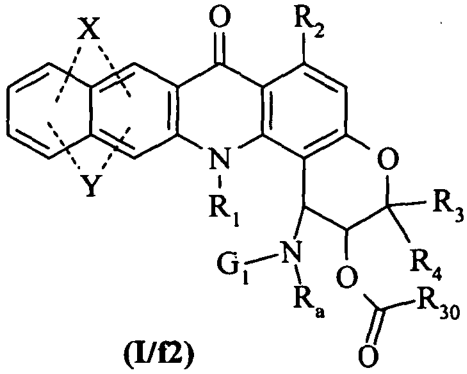

(I/f2)

dans lesquelles X, Y, $R_1$, $R_2$, $R_3$, $R_4$, $R_a$, $G_1$ et $R_{30}$ sont tels que définis précédemment,

❖ soit à des conditions de déshydratation en milieu acide, pour conduire respectivement aux composés de formule (I/e3) et (I/f3), cas particuliers des composés de formule (I) :

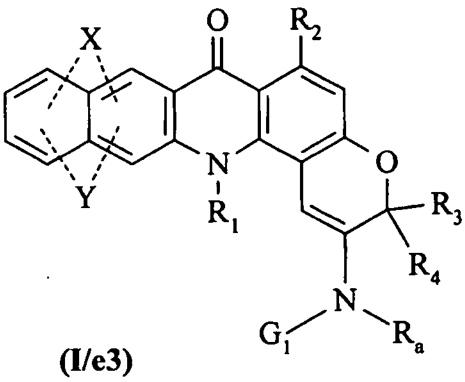

(I/e3)

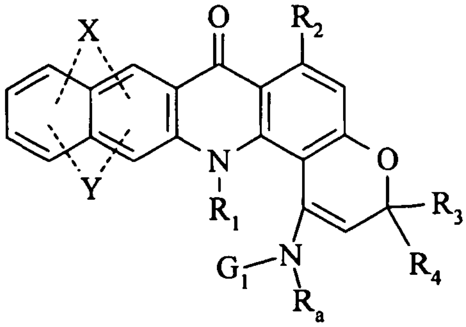

(I/f3)

dans lesquelles X, Y, $R_1$, $R_2$, $R_3$, $R_4$, $R_a$ et $G_1$ sont tels que définis précédemment,

d) ⇒ soit à l'action de $NaN_3$ en présence d'eau oxygénée, suivi d'une étape de réduction par l'hydrure de tri-n-butylétain, par exemple, pour conduire aux composés de formule (XX):

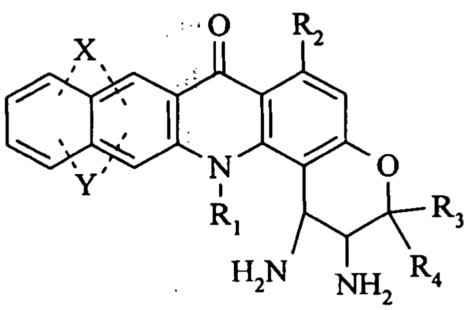

(XX)

dans laquelle X, Y, $R_1$, $R_2$, $R_3$ et $R_4$ sont tels que définis précédemment,
composés de formule (XX) qui sont soumis à l'action d'un composé de formule (XVI) Hal-$G_1$ tel que défini précédemment, pour conduire aux composés de formule (I/g), cas particulier des composés de formule (I) :

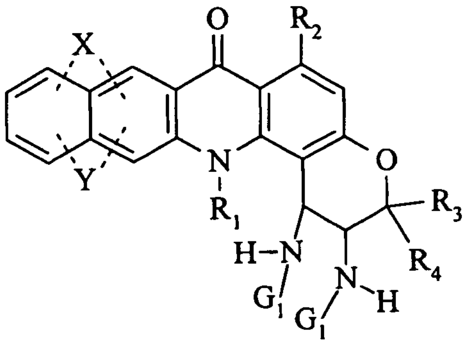

(I/g)

dans laquelle X, Y, $R_1$, $R_2$, $R_3$, $R_4$ et $G_1$ sont tels que définis précédemment,

e) ⇒ soit à l'action du permanganate de potassium en milieu polaire, pour conduire aux composés de formule (XXI) :

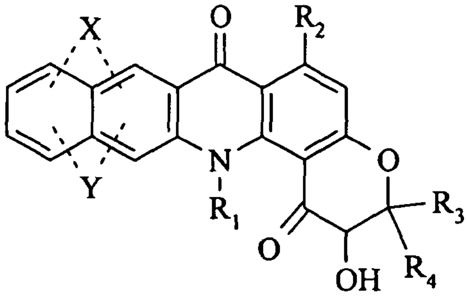

(XXI)

dans laquelle X, Y, $R_1$, $R_2$, $R_3$ et $R_4$ sont tels que définis précédemment,
composés de formule (XXI) qui sont soumis soit à l'action d'un agent alkylant ou d'un agent acylant, pour conduire aux composés de formule (I/h), cas particulier des composés de formule (I) :

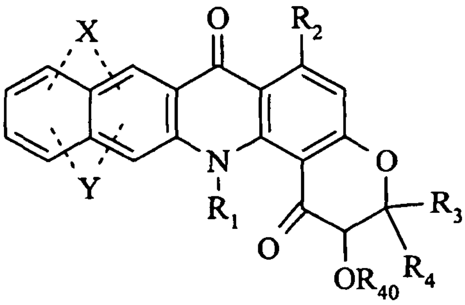

(I/h)

dans laquelle X, Y, $R_1$, $R_2$, $R_3$ et $R_4$ sont tels que définis précédemment, et $R_{40}$ représente un groupement alkyle ($C_1$-$C_6$) linéaire ou ramifié ou un groupement acyle ($C_1$-$C_6$) linéaire ou ramifié,
ou composés de formule (XXI) dont on protège la fonction hydroxy par un groupement protecteur classiquement utilisé en synthèse organique, puis que l'on soumet à l'action de $P_2S_5$, pour conduire aux composés de formule (XXII) :

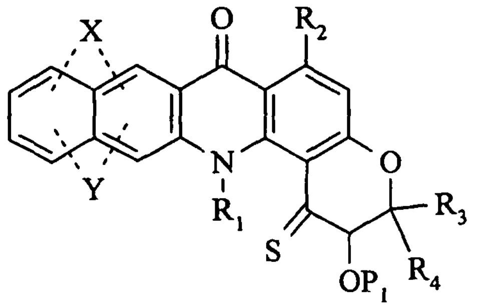

**(XXII)**

dans laquelle X, Y, $R_1$, $R_2$, $R_3$ et $R_4$ sont tels que définis précédemment, et $P_1$ représente un groupement protecteur des fonctions hydroxys,
composés de formule (XXII) qui est traité par un agent de réduction, puis soumis à une réaction de déprotection de la fonction hydroxy, pour conduire aux composés de formule (XXIII) :

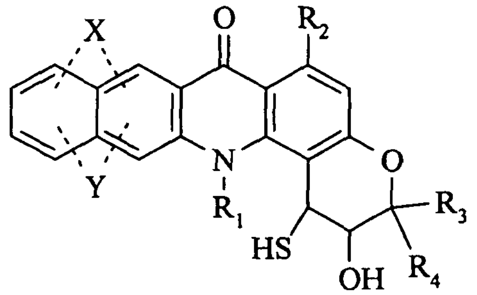

**(XXIII)**

dans laquelle X, Y, $R_1$, $R_2$, $R_3$ et $R_4$ sont tels que définis précédemment,
composés de formule (XXIII) qui est mis en présence d'un composé de formule (XVI) tel que défini précédemment, pour conduire aux composés de formule (I/i), cas particulier des composés de formule (I) :

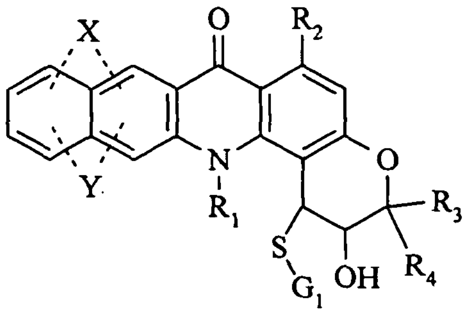

**(I/i)**

dans laquelle X, Y, $G_1$, $R_1$, $R_2$, $R_3$ et $R_4$ sont tels que définis précédemment,
composés de formule (I/i) qui peut être soumis à l'action d'un agent alkylant, d'un agent acylant, ou d'un composé de formule (XVIa) :

**Hal-G'$_1$** **(XVIa)**

dans laquelle Hal représente un atome d'halogène et G'$_1$ représente un groupement choisis parmi -C($W_2$)-$W_3$-$T_1$, -C($W_2$)-$T_1$, -S(O)$_n$-$W_3$-$T_1$ et -S(O)$_n$-$T_1$ dans lesquels $W_2$, $W_3$, $T_1$ et n sont tels que définis dans la formule (I), pour conduire aux composés de formule (I/j), cas particulier des composés de formule (I) :

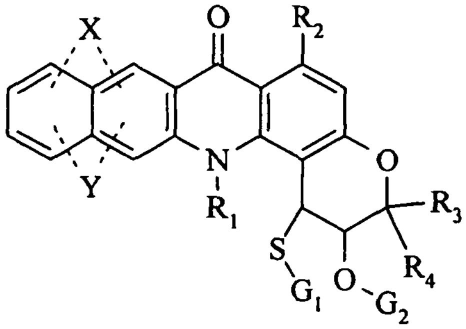

(I/j)

dans laquelle X, Y, $G_1$, $R_1$, $R_2$, $R_3$ et $R_4$ sont tels que définis précédemment et $G_2$ représente un groupement choisi parmi $G'_1$ tel que défini précédemment, alkoxy ($C_1$-$C_6$) linéaire ou ramifié, alkylcarbonyloxy ($C_1$-$C_6$) linéaire ou ramifié, arylcarbonyloxy, arylalkylcarbonyloxy ($C_1$-$C_6$) linéaire ou ramifié,
les composés (I/a) à (I/j), (I/$c_1$) à (I/$c_3$), (I/$d_1$) à (I/$d_3$), (I/$e_1$) à (I/$e_3$), (I/$f_1$) à (I/$f_3$), formant l'ensemble des composés de l'invention que l'on purifie, le cas échéant, selon une technique classique de purification, qui peuvent, si on le désire, être séparés en leurs différents isomères selon une technique classique de séparation et que l'on transforme, si on le souhaite, en leurs N-oxydes et, le cas échéant, en leurs sels d'addition à un acide ou à une base pharmaceutiquement acceptable.

Les composés de formule (VI) peuvent avantageusement être obtenus :

- *soit à partir de dérivés de l'acide 3-amino-2-naphtalène carboxylique (II) :*

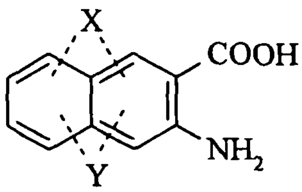

(II)

dans laquelle X et Y sont tels que définis dans la formule (I),
que l'on fait réagir avec un dérivé du phloroglucinol de formule (III) :

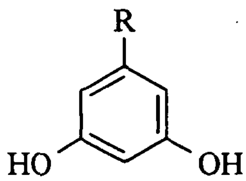

(III)

dans laquelle R représente un atome d'hydrogène, un groupement hydroxy ou un groupement alkyle ($C_1$-$C_6$) linéaire ou ramifié,
pour conduire aux composés de formule (IV) :

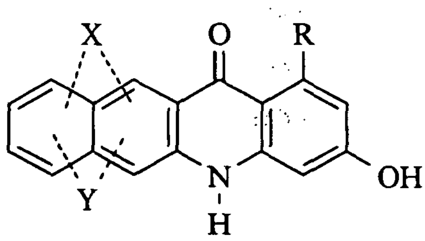

(IV)

dans laquelle X, Y et R sont tels que définis précédemment, qui sont ensuite traités en condition basique dans un solvant aprotique par un alcyne de formule (V) :

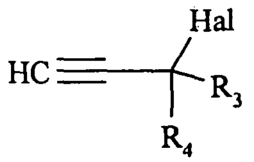

(V)

dans laquelle Hal représente un atome d'halogène et $R_3$ et $R_4$ sont tels que définis dans la formule (I), pour conduire aux composés de formule (VI) tel que défini précédemment,

- *soit à partir de dérivés de l'acide 3-halogéno-2-naphtalène carboxylique de formule (VII) :*

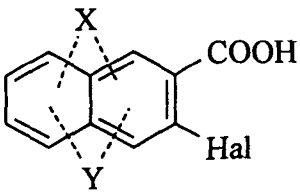

(VII)

dans laquelle X et Y sont tels que définis dans la formule (I) et Hal représente un atome d'halogène, que l'on fait réagir avec un dérivé amino-chromène de formule (VIII) :

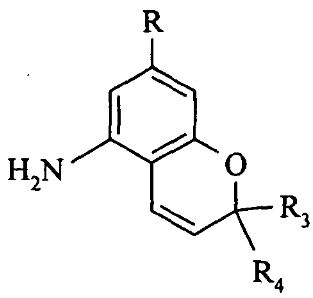

(VIII)

dans laquelle $R_3$ et $R_4$ sont tels que définis dans la formule (I) et R a la même signification que précédemment, pour conduire également aux composés de formule (VI) tel que défini précédemment.

Les composés de formule (XIII) constituent des intermédiaires de synthèse utiles pour l'obtention des composés de formule (I). Parmi ces composés de formule (XIII) on distingue :

- les composés de formule ($IB_1$) :

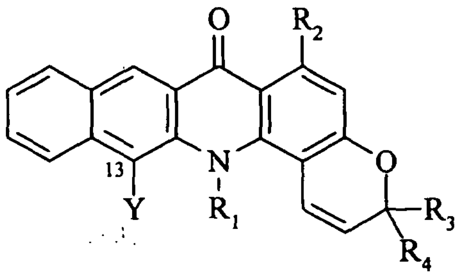

(IB$_1$)

dans laquelle $R_1$, $R_2$, $R_3$ et $R_4$ sont tels que définis dans la formule (IB), et Y représente un groupement amino éventuellement substitué par un ou deux groupements, identiques ou différents, indépendamment l'un de l'autre, choisis parmi alkyle ($C_1$-$C_6$) linéaire ou ramifié, acyle ($C_1$-$C_6$) linéaire ou ramifié, ou trihalogénoalkylcarbonyle ($C_1$-$C_6$) linéaire ou ramifié,

➤ et les composés de formule (IB$_2$) :

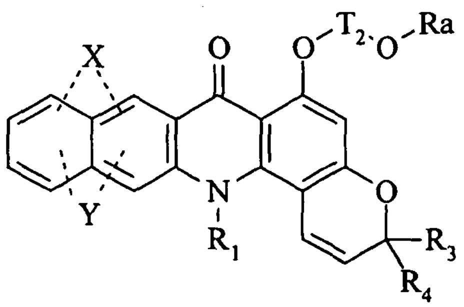

(IB$_2$)

dans laquelle $R_1$, $R_3$, $R_4$, $T_2$, Ra, X et Y sont tels que définis dans la formule (I).

Ces composés de formule (IB$_1$) et (IB$_2$) sont nouveaux et possèdent une activité cytotoxique. Ils sont donc utiles dans le traitement des cancers.

Les composés de formule (XXI) constituent également des intermédiaires de synthèse utiles pour l'obtention des composés de formule (I) et plus particulièrement des composés de formule (IC). Ces composés de formule (XXI) possèdent également une activité cytotoxique. Leur utilisation en tant que principe actif au sein de composition pharmaceutique rend cette dernière utile pour le traitement des cancers.

Les composés de formules (II), (III), (V), (VII), (VIII), (XI), (XIV) et (XVI) sont soit des produits commerciaux, soit obtenus selon des méthodes classiques de la synthèse organique bien connues de l'homme du métier.

Les composés de formule (I) présentent des propriétés antitumorales particulièrement intéressantes. Ils ont un excellente cytotoxicité in vitro sur des lignées cellulaires, issues de tumeurs murines et humaines, due à un blocage spécifique du cycle cellulaire, et sont actifs in vivo, chez la souris, sur des tumeurs transplantables murines et humaines. Les propriétés caractéristiques de ces composés permettent leur utilisation en thérapeutique en tant qu'agents antitumoraux.

La présente invention a également pour objet les compositions pharmaceutiques contenant comme principe actif au moins un composé de formule (I), ses énantiomères, diastéréoisomères, N-oxydes ou un de ses sels d'addition à une base ou un acide pharmaceutiquement acceptable, seul ou en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire, ou sous-cutanée), per ou trans-cutanée, intravaginale, rectale, nasale, perlinguale, buccale, oculaire ou respiratoire.

Les compositions pharmaceutiques selon l'invention pour les injections parentérales comprennent notamment les solutions stériles aqueuses et non aqueuses, les dispersions, les suspensions ou émulsions ainsi que les poudres stériles pour la reconstitution des solutions ou des dispersions injectables.

Les compositions pharmaceutiques selon l'invention, pour les administrations orales solides, comprennent notamment les comprimés simples ou dragéifiés, les comprimés sublinguaux, les sachets, les gélules, les granules, et

pour les administrations liquides orales, nasales, buccales ou oculaires, comprennent notamment les émulsions, les solutions, les suspensions, les gouttes, les sirops et les aérosols.

Les compositions pharmaceutiques pour l'administration rectale ou vaginale sont préférentiellement des suppositoires, et celles pour l'administration per ou trans-cutanée comprennent notamment les poudres, les aérosols, les crèmes, les pommades, les gels et les patchs.

Les compositions pharmaceutiques citées précédemment illustrent l'invention mais ne la limitent en aucune façon.

Parmi les excipients ou véhicules inertes, non toxiques, pharmaceutiquement acceptables, on peut citer à titre indicatif et non limitatif les diluants, les solvants, les conservateurs, les agents mouillants, les émulsifiants, les agents dispersants, les liants, les agents gonflants, les agents désintégrants, les retardants, les lubrifiants, les absorbants, les agents de suspension, les colorants, les aromatisants, etc...

La posologie utile varie selon l'âge et le poids du patient, la voie d'administration, la composition pharmaceutique utilisée, la nature et la sévérité de l'affection, et la prise de traitements associés éventuels. La posologie s'échelonne de 0,1 mg à 1000 mg en une ou plusieurs prises par jour.

Les exemples suivants illustrent l'invention mais ne la limitent en aucune façon.

Les produits de départ utilisés sont des produits connus ou préparés selon des modes opératoires connus. Les différentes préparations conduisent à des intermédiaires de synthèse utiles pour la préparation des composés de l'invention.

Les structures des composés décrits dans les exemples et dans les préparations ont été déterminées selon les techniques spectrophotométriques usuelles (infrarouge, résonance magnétique nucléaire, spectrométrie de masse, ...).

Les points de fusion ont été déterminés soit à la platine chauffante de Kofler, soit à la platine chauffante sous microscope. Lorsque le composé existe sous forme de sel, le point de fusion correspond à celui du produit salifié.

**<u>Préparation 2</u> : (±)*cis*-1,2-Dihydroxy-6-méthoxy-3,3,14-triméthyl-2,3,7,14-tétrahydro -1*H*-benzo[*b*]pyrano [3,2-*h*]acridin-7-one**

A une solution de 2 g du produit de la préparation 1 et de 0,9 g de 4-méthylmorpholine-N-oxyde monohydrate dans 40 ml d'un mélange t-butanol/tétrahydrofurane/eau (10/3/1) est additionné 2,5 % de tétroxyde d'osmium en solution dans 3,8 ml de 2-méthyl-2-propanol. Après 2 jours à température ambiante, 105 ml d'une solution saturée en $NaHSO_3$ sont ajoutés et le milieu réactionnel est maintenu 1 heure sous agitation, puis extrait au dichlorométhane. Les phases organiques rassemblées sont séchées sur sulfate de sodium et concentrées sous vide. Une chromatographie sur gel de silice (dichlorométhane/méthanol : 95/5) permet d'isoler 1,3 g du produit attendu.

*<u>Point de fusion :</u> 194 °C.*

**<u>Préparation 5</u> : (±)-*cis*-1,2-Dihydroxy-6-(diéthylaminopropylamino)-3,3,14-triméthyl-2,3,7,14-tétrahydro-1*H*-benzo[*b*]pyrano[3,2-*h*]acridin-7one**

On procède comme dans la préparation 2 en utilisant comme substrat le composé de la préparation 3.

**<u>Préparation 6</u> : (±)-1-Amino-2-hydroxy-6-méthoxy-3,3,14-triméthyl-2,3,7,14-tétrahydro-1*H*-benzo[*b*]pyrano [3,2-*h*]acridin-7-one**

*<u>Stade A</u> : (±)-1-Azido-2-hydroxy-6-méthoxy-3, 3,14-triméthyl-2,3,7,14-tétrahydro-1H-benzo[b]pyrano[3,2-h]acridin-7-one*

A une solution de 0,15 g du composé de la préparation 2, et 0,5 g de $NaN_3$ dans 6 ml de chloroforme, sont additionnés lentement à température ambiante 3 ml d'acide trifluoroacétique. Après 12 heures d'agitation, 1 équivalent de $NaN_3$ est ajouté et la réaction est maintenue à température ambiante pendant encore 12 heures. Le mélange réactionnel est ensuite lavé à l'eau puis par une solution saturée en NaCl, séché sur sulfate de sodium. Une chromatographie sur gel de silice (dichlorométhane/méthanol : 95/5) permet d'isoler le produit attendu.

*<u>Stade B</u> : (±)-1-Amino-2-hydroxy-6-méthoxy-3,3,14-triméthyl-2,3,7,14-tétrahydro-1H-benzo[b]pyrano[3,2-h]acridin-7-one.*

Une solution contenant 0,2 g du composé obtenu au stade A et 0,09 g de Pd/C dans 5 ml d'éthanol est agitée à température ambiante sous atmosphère d'$H_2$ pendant 48 heures. Le catalyseur est ensuite filtré et le filtrat est concentré sous vide. Le résidu est chromatographié sur gel de silice (dichlorométhane/méthanol : 95/5) permettant d'isoler le

produit désiré.

**Préparation 8 : 1,2-Diamino-6-méthoxy-3,3,14-triméthyl-2,3,7,14-tétrahydro-1*H*-benzo[*b*]pyrano[3,2-*h*]acridin-7-one**

Une chromatographie sur gel de silice permet d'isoler ce co-produit qui se forme lors de la synthèse du composé de la préparation 6.

**Exemple 7 : (±)-cis-1,2-Dipentènoyloxy-6-méthoxy-3,3,14-triméthyl-2,3,7,14-tétrahydro-1*H* benzo[*b*]pvrano [3,2-*h*]acridin-7-one.**

Une solution de 1,48 mmol du composé de la préparation 2 dans 15 ml de pyridine anhydre est additionnée à 14,8 mmol de chlorure de pentènoyle. Le milieu réactionnel est maintenu sous agitation à température ambiante pendant 6 heures puis extrait au dichlorométhane. Les phases organiques réunies sont séchées sur sulfate de magnésium, filtrées puis évaporées sous pression réduite. Une chromatographie sur gel de silice du résidu (dichlorométhane/méthanol : gradient de 0,2 à 2%) permet d'isoler le produit attendu.
*Point de fusion :* *152°C*

**Exemple 8 : (±)-cis-1-Hydroxy-6-méthoxy-2-pentènoyloxy-3,3,14-triméthyl-2,3,7,14-tétrahydro-1*H*-benzo[*b*]pyrano[3,2-*h*]acridin-7-one**

Le co-produit est isolé lors de la chromatographie sur gel de silice du composé de l'exemple 7.
*Point de fusion :* *194°C*

**Exemple 11 : (1S,2S)-1-{[(Diméthylamino)carbonyl]oxy}-6-méthoxy-3,3,14-triméthyl-7-oxo-2,3,7,14-tétrahydro-1*H*-benzo[*b*]pyrano[3,2-*h*]acridin-2-yl diméthylcarbamate**

Dans un ballon, 0,698 mmol d'hydrure de potassium est lavé trois fois à l'hexane puis refroidi à -10°C, et une solution de 0,123 mmol du composé de la préparation 2 dans 4 ml de tétrahydrofurane anhydre est ajoutée. Après addition goutte à goutte à -10°C de 0,327 mmol de chlorure de *N,N*-diméthylcarbamoyle, l'agitation est maintenue 3h30 à température ambiante. La réaction est arrêtée par addition de 50 ml d'acétate d'éthyle et de 10 ml d'une solution saturée en $NaHCO_3$ afin d'obtenir un pH de 8. La phase organique est lavée à l'eau et séchée sur sulfate de magnésium. Après évaporation sous pression réduite et cristallisation dans l'acétate d'éthyle le produit attendu est isolé.
*Point de fusion :* *173°C*

**Exemple 13 : (1S,2S)-6-{[3-(Diéthylamino)propyl]amino}-1-{[(diméthylamino) carbonyl]oxy}-3,3,14-triméthyl-7-oxo-2,3,7,14-tétrahydro-1*H*-benzo[*b*]pyrano[3,2-*h*]acridin-2-yl diméthylcarbamate**

Le produit est obtenu selon le procédé de l'exemple 11, des stades 1 à 2, en utilisant comme substrat le produit de la préparation 5.

**Exemple 14 : (1S,2S)-1-{[(Diméthylamino)carbonyl]amino}-6·méthoxy-3,3,14-triméthyl-7-oxo-2,3,7,14-tétrahydro-1*H*-benzo[*b*]pyrano[3,2-*h*]acridin-2-yl diméthylcarbamate**

Le produit est obtenu selon le procédé de l'exemple 11, des stades 1 à 2, en utilisant comme substrat le produit de la préparation 6.

**Exemple 15 : *N*'-((1S,2S)-1-{[(Diméthylamino)carbothioyl]amino}-6-méthoxy-3,3,14-triméthyl-7-oxo-2,3,7,14-tétrahydro-1*H*-benzo[*b*]pyrano[3,2-*h*]acridin-2-yl)-*N,N*-diméthylthiourée**

Le produit est obtenu selon le procédé de l'exemple 11, des stades 1 à 2, en utilisant comme substrat le produit de la préparation 8 et comme réactif le chlorure de diméthylthiocarbamoyle.

**Exemple 23 : (±)-cis-1-{[(Diéthylamino)carbonyl]oxy}-6-méthoxy-3,3,14-triméthyl-7-oxo-2,3,7,14-tétrahydro-1*H*-benzo[*b*]pyrano[3,2-*h*]acridin-2-yl diéthylcarbamate**

Le produit est obtenu selon le procédé de l'exemple 11 en utilisant comme réactif le chlorure de *N,N*-diéthylcarbamoyle.

*Point de fusion : 193°C*

**Exemple 24 : (±)-cis-1-{[(Diisopronylamino)carbonyl]oxyl}-6-méthoxy-3,3,14-triméthyl-7-oxo-2,3,7,14-tétrahydro-1*H* benzo[*b*]pyrano[3,2-*h*]acridin-2-yl diisopropylcarbamate**

Le produit est obtenu selon le procédé de l'exemple 11 en utilisant comme réactif le chlorure de NN-diisopropylcarbamoyle.
*Point de fusion : 149,5°C*

**Exemple 25 : (±)-cis-1-{[(Dibutylamino)carbonyl]oxy}-6-méthoxy-3,3,14-triméthyl-7-oxo-2,37,14-tétrahydro-1*H*-benzo[*b*]pyrano[3,2-*h*]acridin-2-yl dibutylcarbamate**

Le produit est obtenu selon le procédé de l'exemple 11 en utilisant comme réactif le chlorure de *N,N*-dibutylcarbamoyle.
*Point de fusion : 138°C*

***ETUDE PHARMAGOLOGIOUE DES COMPOSES DE L'INVENTION***

**EXEMPLE 27 : Activité in vitro**

La leucémie murine L1210 a été utilisée in vitro. Les cellules sont cultivées dans du milieu de culture RPMI 1640 complet contenant 10 % de sérum de veau foetal, 2 mM de glutamine, 50 U/ml de pénicilline, 50 µg/ml de streptomycine et 10 mM d'Hepes, pH : 7,4. Les cellules sont réparties dans des microplaques et exposées aux composés cytotoxiques pendant 4 temps de doublement, soit 48 heures. Le nombre de cellules viables est ensuite quantifié par un essai colorimétrique, le Microculture Tetrazolium Assay (J. Carmichael et al., Cancer Res., 47, 936-942, (1987)). Les résultats sont exprimés en $IC_{50}$, concentration en cytotoxique qui inhibe à 50 % la prolifération des cellules traitées.
A titre d'exemple, les composés des exemples 11 et 7 montrent respectivement un $IC_{50}$ de 0,78 µM et 0,52 µM, démontrant ainsi leur plus grande activité que le composé de référence, l'acronycine.

**EXEMPLE 28: Activité in vivo**

***1- Activité antitumorale sur la leucémie P 388***

La lignée P388 (leucémie murine) a été fournie par le National Cancer Institute (Frederick, USA). Les cellules tumorales ($10^6$ cellules) ont été inoculées au jour 0 dans la cavité péritonéale de souris B6D2F1 femelles (Iffa Credo, France). Six souris de 18 à 20 g ont été utilisées par groupe expérimental. Les produits ont été administrés par voie intrapéritonéale au jour 1.
L'activité antitumorale est exprimée en % de T/C :

$$T/C\ \%\ (\text{souris}) = \frac{\text{Temps de survie médian des animaux traités}}{\text{Temps de survie médian des animaux contrôles}} \times 100$$

Les composés de la présente invention sont très actif dans ce modèle avec un T/C >150%, alors que l'acronycine n'est que marginalement active.

***2- Activité antitumorale sur l'adénocarcinome du colon C38***

Les fragments tumoraux d'adénocarcinome du colon C38, pesant environ 30 mg, ont été implantés au jour 0 sous la peau de souris B6D2F 1 (Iffa Credo, France).
Après croissance de la tumeur, les souris ont été séparées en groupes contrôle (18 animaux) et traité (6 à 7 animaux), homogènes quant à la taille tumorale. Les produits ont été administrés par voie i.v. une fois par semaine pendant 3 semaines (aux jours 10, 17 et 24), à leur Dose Maximale Tolérée (MTD), MTD/2 et MTD/4.
Les tumeurs ont été mesurées deux fois par semaine et les volumes tumoraux ont été calculés suivant la formule : Volume ($mm^3$) = longueur (mm) x largeur ($mm^2$) / 2.
L'activité antitumorale est exprimée en % de T/C :

$$\% \, T/C = \frac{\text{Vt/V0 médian des animaux traités}}{\text{Vt/V0 médian des animaux contrôle}} \times 100$$

V0 et Vt étant respectivement le volume initial de la tumeur et son volume au temps de mesure t.

La dose optimale est la dose qui donne le T/C le plus bas sans toxicité (mort prématurée ou perte de poids supérieure à 20%).

A titre d'exemple, les composés des exemples 7 et 11 montrent une activité antitumorale de 30% pour une dose optimale de 12.5 mg/kg et 41 % pour une dose optimale de 25 mg/kg respectivement alors que l'acronycine montre une activité antitumorale de 27% pour une dose optimale de 100 mg/kg permettant de démontrer leur fort potentiel thérapeutique.

**EXEMPLE 29 : Composition pharmaceutique: soluté injectable**

| | |
|---|---|
| Composé de l'exemple 11 | 10 mg |
| Eau distillée pour préparations injectables | 25 ml |

## Revendications

**1.** Composés de formule (I) :

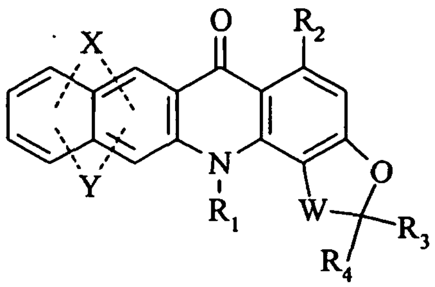

**(I)**

dans laquelle :

- **X** et **Y,** identiques ou différents, indépendamment l'un de l'autre, représentent chacun un groupement choisi parmi :

  - atome d'hydrogène, d'halogène,
  - groupement mercapto, cyano, nitro, alkyle ($C_1$-$C_6$) linéaire ou ramifié, trihalogénoalkyle ($C_1$-$C_6$) linéaire ou ramifié, trihalogénoalkylcarbonylamino ($C_1$-$C_6$) linéaire ou ramifié,
  - groupement de formules ORa, NRaRb, -NRa-C(O)-$T_1$, -O-C(O)-$T_1$, -O-$T_2$-NRaRb, -O-$T_2$-ORa, -NRa-$T_2$-NRaRb, -NRa-$T_2$-ORa, et -NRa-$T_2$-$CO_2$Ra dans lesquels :

    * **Ra** représente un groupement choisi parmi atome d'hydrogène, groupement alkyle ($C_1$-$C_6$) linéaire ou ramifié, aryle, et arylalkyle ($C_1$-$C_6$) linéaire ou ramifié,
    * **Rb** représente un groupement choisi parmi atome d'hydrogène, groupement alkyle ($C_1$-$C_6$) linéaire ou ramifié, aryle, et arylalkyle ($C_1$-$C_6$) linéaire ou ramifié,
    ou
    **Ra+Rb** forment ensemble, avec l'atome d'azote qui les portent, un hétérocycle de 5 à 6 chaînons, monocyclique, contenant éventuellement au sein du système cyclique un second hétéroatome choisi parmi oxygène et azote,
    * **$T_1$** représente un groupement choisi parmi alkyle ($C_1$-$C_6$) linéaire ou ramifié, alkènyle ($C_2$-$C_6$) linéaire

ou ramifié, aryle, arylalkyle ($C_1$-$C_6$) linéaire ou ramifié, et une chaîne alkylène ($C_1$-$C_6$) linéaire ou ramifié substituée par un groupement choisi parmi -ORa et -NRaRb dans lesquels Ra et Rb sont tels que définis précédemment,

* **$T_2$** représente une chaîne alkylène ($C_1$-$C_6$) linéaire ou ramifiée,

- ou X et Y, lorsqu'ils sont en positions adjacentes, forment ensemble un groupement méthylènedioxy ou un groupement éthylènedioxy,

étant entendu que les substituants X et Y peuvent être présents sur l'un ou l'autre des deux cycles benzéniques adjacents,

• **$R_1$** représente un atome d'hydrogène ou un groupement alkyle ($C_1$-$C_6$) linéaire ou ramifié,

• **$R_2$** représente un groupement choisi parmi atome d'hydrogène, groupement alkyle ($C_1$-$C_6$) linéaire ou ramifié, ORa, NRaRb, -NRa-C(O)-$T_1$, -O-C(O)-$T_1$, -O-$T_2$-NRaRb, -O-$T_2$-ORa, -NRa-$T_2$-NRaRb, -NRa-$T_2$-ORa, et -NRa-$T_2$-$CO_2$Ra dans lesquels Ra, Rb, $T_1$ et $T_2$ sont tels que définis précédemment,

• **$R_3$, $R_4$,** identiques ou différents, indépendamment l'un de l'autre, représentent un atome d'hydrogène ou un groupement alkyle ($C_1$-$C_6$) linéaire ou ramifié,

• **W** représente un groupement de formule -CH($R_5$)-CH($R_6$)- dans laquelle:

* **$R_5$** et/ou **$R_6$,** représente(nt) un groupement choisi parmi -$W_1$-C($W_2$)-$W_3$-$T_1$, -$W_1$-S(O)$_n$-$W_3$-$T_1$ et -$W_1$-S(O)$_n$-$T_1$ dans lesquels :

- $W_1$ représente un atome d'oxygène, de soufre ou un atome d'azote substitué par un atome d'hydrogène, un groupement alkyle ($C_1$-$C_6$) linéaire ou ramifié, aryle ou arylalkyle ($C_1$-$C_6$) linéaire ou ramifié,
- $W_2$ représente un atome d'oxygène ou un atome de soufre,
- $W_3$ représente un atome d'oxygène, de soufre ou un atome d'azote substitué par un atome d'hydrogène, un groupement alkyle ($C_1$-$C_6$) linéaire ou ramifié, aryle ou arylalkyle ($C_1$-$C_6$) linéaire ou ramifié, une liaison lorsque $T_1$ représente un groupement alkényle $C_4$,
- $T_1$ est tel que défini précédemment,
- n représente un entier choisi parmi 1 ou 2,

et dans le cas où un seul des deux groupements $R_5$ et $R_6$ représente un groupement tel que défini précédemment, alors l'autre desdits groupements $R_5$ ou $R_6$ représente un groupement choisi parmi atome d'hydrogène, hydroxy, alkoxy ($C_1$-$C_6$) linéaire ou ramifié, alkylcarbonyloxy ($C_1$-$C_6$) linéaire ou ramifié, arylcarbonyloxy, arylalkylcarbonyloxy ($C_1$-$C_6$) linéaire ou ramifié, et amino éventuellement substitué par un ou deux groupements alkyle ($C_1$-$C_6$) linéaire ou ramifié, identiques ou différents,

leurs énantiomères, diastéréoisomères, N-oxydes, ainsi que leurs sels d'addition à un acide ou à une base pharmaceutiquement acceptable,

étant entendu que par aryle, on comprend un groupement phényle ou naphtyle, comportant éventuellement une ou plusieurs substitutions, identiques ou différentes, choisies parmi hydroxy, halogène, carboxy, nitro, amino, alkylamino ou dialkylamino ($C_1$-$C_6$) linéaire ou ramifié, alkoxy ($C_1$-$C_6$) linéaire ou ramifié, acyle ($C_1$-$C_6$) linéaire ou ramifié, et alkylcarbonyloxy ($C_1$-$C_6$) linéaire ou ramifié, et que par isomère on comprend les isomères optiques.

2. Composés de formule (I) selon la revendication 1 **caractérisés en ce que** $R_5$ et $R_6$, identiques, représentent un groupement de formule -$W_1$-C($W_2$)-$W_3$-$T_1$ ou -$W_1$-S(O)$_n$-$T_1$ dans laquelle $W_1$, $W_2$, $W_3$, $T_1$ et n sont tels que définis dans la formule (I), leurs énantiomères, diastéréoisomères, N-oxydes, ainsi que leurs sels d'addition à un acide ou à une base pharmaceutiquement acceptable.

3. Composés de formule (I) selon l'une quelconque des revendications 1 à 2, **caractérisés en ce que** $R_5$ et $R_6$, identiques, représentent un groupement de formule -$W_1$-C($W_2$)-$W_3$-$T_1$ dans laquelle $W_1$ représente un atome d'oxygène, $W_2$ représente un atome d'oxygène, $W_3$ représente un atome d'azote substitué par un atome d'hydrogène, un groupement alkyle ($C_1$-$C_6$) linéaire ou ramifié, aryle ou arylalkyle ($C_1$-$C_6$) linéaire ou ramifié et $T_1$ est tel que défini dans la formule (I), leurs énantiomères, diastéréoisomères, N-oxydes, ainsi que leurs sels d'addition à un acide ou à une base pharmaceutiquement acceptable.

4. Composés de formule (I) selon l'une quelconque des revendications 1 à 2, **caractérisés en ce que** $R_5$ et $R_6$, identiques, représentent un groupement de formule -$W_1$-S(O)$_n$-$T_1$ dans laquelle $W_1$ représente un atome d'oxygène, $T_1$ est tel que défini dans la formule (I) et n est égal à 2, leurs énantiomères, diastéréoisomères, N-oxydes, ainsi que leurs sels d'addition à un acide ou à une base pharmaceutiquement acceptable.

**5.** Composés de formule (I) selon l'une quelconque des revendications 1 à 4 **caractérisés en ce que** $R_3$ et $R_4$, identiques ou différents, représentent un groupement alkyle ($C_1$-$C_6$) linéaire ou ramifié, leurs énantiomères, diastéréoisomères, N-oxydes, ainsi que leurs sels d'addition à un acide ou à une base pharmaceutiquement acceptable.

**6.** Composés de formule (I) selon l'une quelconque des revendications 1 à 4, **caractérisés en ce que** $R_2$ représente un groupement choisi parmi alkoxy ($C_1$-$C_6$) linéaire ou ramifié, NRaRb, -O-$T_2$-NRaRb, -O-$T_2$-ORa, -NRa-$T_2$-NRaRb et -NRa-$T_2$-ORa dans lesquels Ra, Rb, $T_1$ et $T_2$ sont tels que définis dans la formule (I), leurs énantiomères, diastéréoisomères, N-oxydes, ainsi que leurs sels d'addition à un acide ou à une base pharmaceutiquement acceptable.

**7.** Composés de formule (I) selon la revendication 1 qui sont le :

- (1*S*,2*S*)-1-{[(diméthylamino)carbonyl]oxy}-6-méthoxy-3,3,14-triméthyl-7-oxo-2,3,7,14-tétrahydro-1*H*-benzo[*b*]pyrano[3,2-*h*]acridin-2-yl diméthylcarbamate
- et le (1*S*,2*S*)-6-méthoxy-3,3,14-triméthyl-2-{[(4-méthylphényl)sulfonyl]oxy}-7-oxo-2,3,7,14-tétrahydro-1*H*-benzo[*b*]pyrano[3,2-*h*]acridin-1-yl 4-méthylbenzènesulfonate,

leurs énantiomères, diastéréoisomères, N-oxydes, ainsi que leurs sels d'addition à un acide ou à une base pharmaceutiquement acceptable.

**8.** Procédé de préparation des composés de formule (I) selon la revendication 1 **caractérisé en ce** en ce qu'on utilise comme produit de départ un composé de formule (VI) :

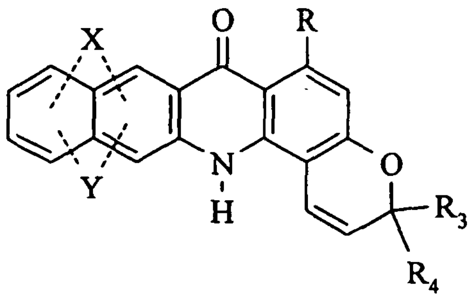

(VI)

dans laquelle X, Y, R, $R_3$ et $R_4$ sont tels que définis dans la formule (I),
composé de formule (VI) dont l'atome d'azote est éventuellement substitué, par action d'un halogénure d'alkyle ou d'un sulfate de dialkyle en présence d'un agent de déprotonation, en solvant polaire aprotique ou en condition de transfert de phase, permettant d'obtenir les composés de formule (IX) :

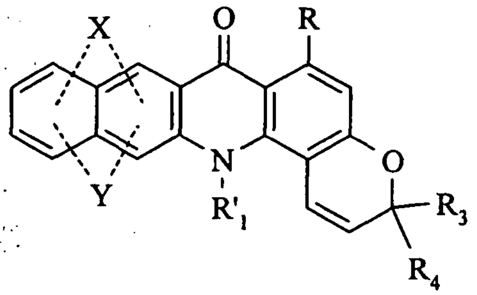

(IX)

dans laquelle X, Y, R, $R_3$ et $R_4$ sont tels que définis précédemment, et $R'_1$ représente un groupement alkyle ($C_1$-$C_6$) linéaire ou ramifié,
composés de formule (IX) qui sont soumis à l'action d'un agent alkylant ou d'un agent acylant selon des conditions classiques de la synthèse organique, pour conduire aux composés de formule (X) :

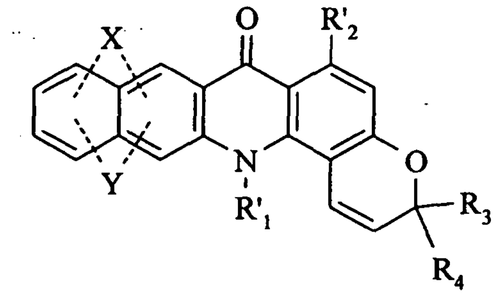

**(X)**

dans laquelle X, Y, $R'_1$, $R_3$ et $R_4$ sont tels que définis précédemment et $R'_2$ représente un groupement choisi parmi ORa, -O-C(O)-$T_1$, -O-$T_2$-NRaRb, -O-$T_2$-ORa dans lesquels Ra, Rb, $T_1$ et $T_2$ sont tels que définis dans la formule (I), composés de formule (X) qui sont traités, dans le cas où $R'_2$ représente un groupement alkoxy par un composé de formule (XI) :

$HNRaR_{10}$ **(XI)**

dans laquelle Ra représente un atome d'hydrogène, un groupement alkyle ($C_1$-$C_6$) linéaire ou ramifié, aryle, ou arylalkyle ($C_1$-$C_6$) linéaire ou ramifié, et $R_{10}$ représente un groupement choisi parmi Rb, -C(O)-$T_1$, -$T_2$-NRaRb, -$T_2$-ORa, et -$T_2$-$CO_2$Ra dans lesquels Ra, Rb, $T_1$ et $T_2$ sont tels que définis dans la formule (I), pour conduire aux composés de formule (XII) :

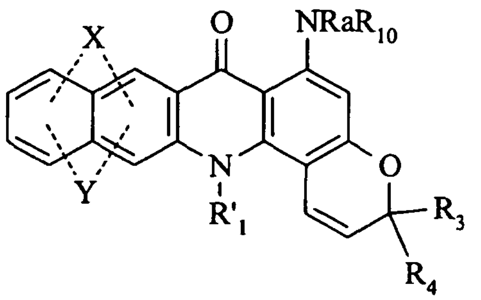

**(XII)**

dans laquelle X, Y, $R'_1$, $R_3$, $R_4$, Ra et $R_{10}$ sont tels que définis précédemment,
l'ensemble des composés de formule (VI), (IX), (X) et (XII) formant les composés de 0formule (XIII) :

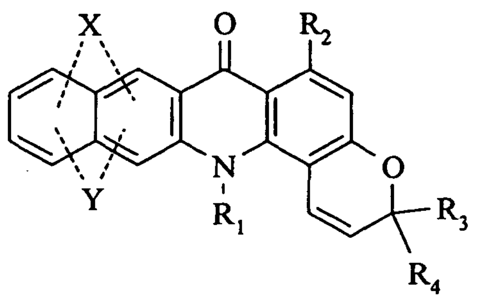

**(XIII)**

dans laquelle X, Y, $R_1$, $R_2$, $R_3$ et $R_4$ ont la même signification que dans la définition générale de la formule (I), ***composés de formule (XIII) qui sont soumis :***

a) ⇒ soit à l'action d'un agent oxydant des fonctions alcènes, pour conduire aux composés de formule (XV) :

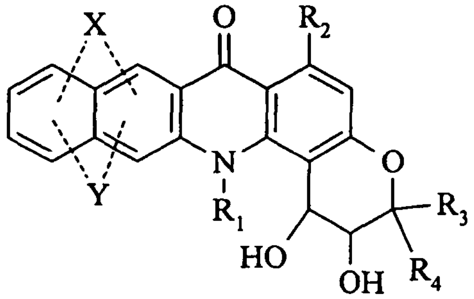

(XV)

dans laquelle X, Y, $R_1$, $R_2$, $R_3$ et $R_4$ sont tels que définis précédemment,
composés de formule (XV) qui sont traités par un composé de formule (XVI) :

**Hal-$G_1$** **(XVI)**

dans laquelle Hal représente un atome d'halogène et $G_1$ représente un groupement choisi parmi $-C(W_2)-W_3-T_1$, $-C(W_2)-T_1$, $-S(O)_n-W_3-T_1$ et $-S(O)_n-T_1$ dans lesquels $W_2$, $W_3$, $T_1$ et n sont tels que définis dans la formule (I), pour conduire aux composés de formules (I/b), (I/c) et (I/d), cas particulier des composés de formule (I) :

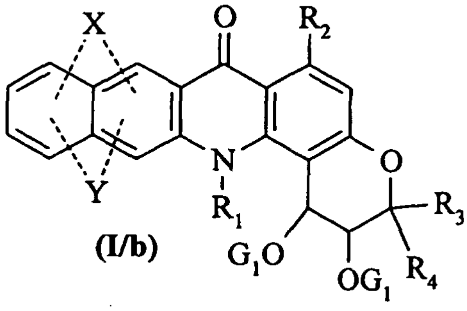

(I/b)

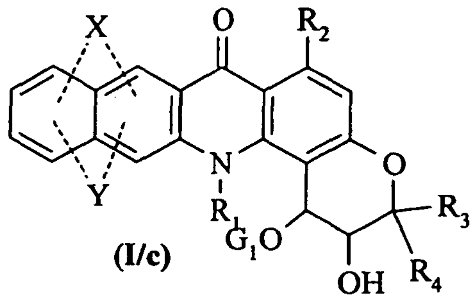

(I/c)

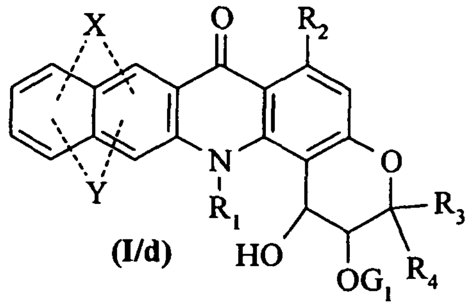

(I/d)

dans laquelle X, Y, $R_1$, $R_2$, $R_3$, $R_4$ et $G_1$ sont tels que définis précédemment,
composés de formule (I/c) et/ou (I/d) qui sont soumis :

❖ soit à l'action d'un alcool de formule $R_{20}$-OH dans laquelle $R_{20}$ représente un groupement alkyle ($C_1$-$C_6$) linéaire ou ramifié, pour conduire respectivement aux composés de formule (I/c1) et (1/d1):

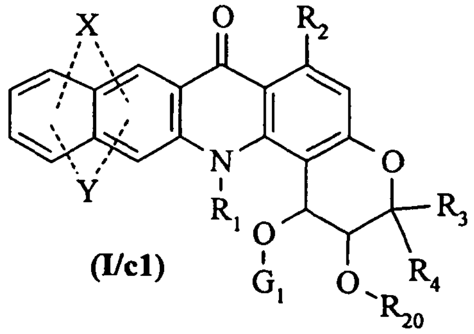

(I/c1)

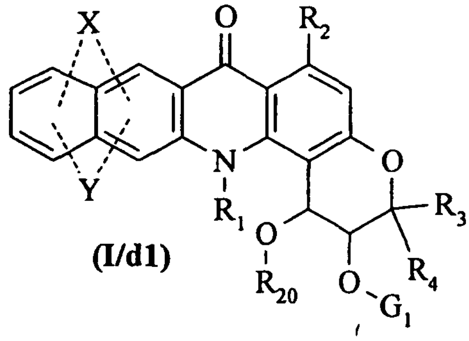

(I/d1)

dans laquelle X, Y, $R_1$, $R_2$, $R_3$, $R_4$, $G_1$ et $R_{20}$ sont tels que définis précédemment,

❖ soit à l'action d'un anhydride de formule $(R_{30}CO)_2O$ dans laquelle $R_{30}$ représente un groupement alkyle ($C_1$-$C_6$) linéaire ou ramifié, aryle ou arylalkyle ($C_1$-$C_6$) linéaire ou ramifié, pour conduire respectivement aux composés de formule (I/c2) et (I/d2) :

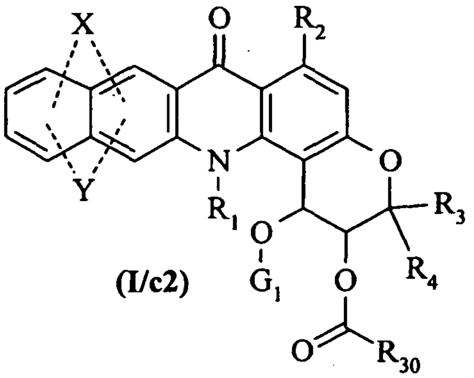

(I/c2)

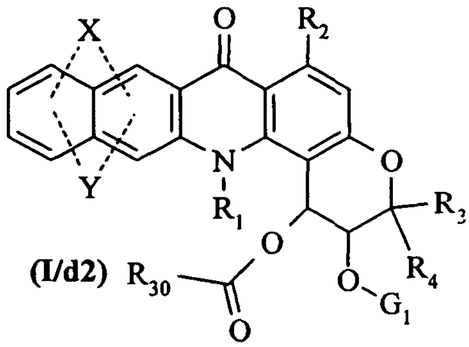

(I/d2)

dans laquelle X, Y, $R_1$, $R_2$, $R_3$, $R_4$, $G_1$ et $R_{30}$ sont tels que définis précédemment,

❖ soit à des conditions de déshydratation en milieu acide, pour conduire respectivement aux composés de formule (I/c3) et (I/d3), cas particulier des composés de formule (I) :

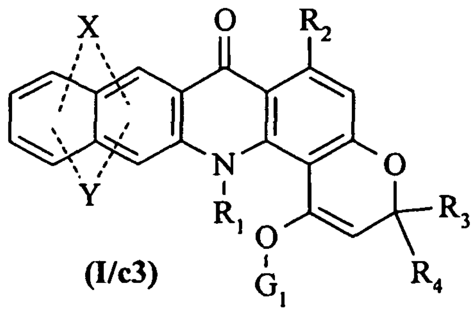

(I/c3)

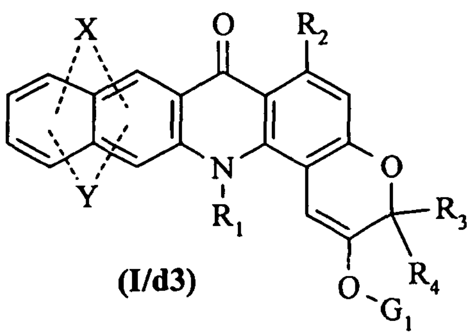

(I/d3)

dans laquelle X, Y, $R_1$, $R_2$, $R_3$, $R_4$ et $G_1$ sont tels que définis précédemment,

b) ⇒ soit à l'action d'un peracide ou du diméthyle dioxirane, pour conduire aux composés de formule (XVII) :

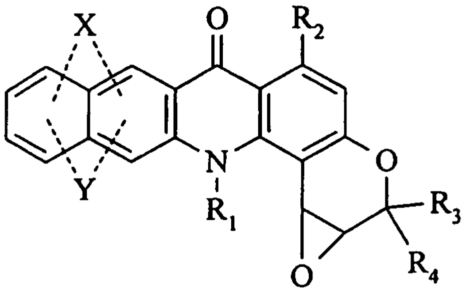

(XVII)

dans laquelle X, Y, $R_1$, $R_2$, $R_3$ et $R_4$ sont tels que définis précédemment,

composés de formule (XVII) qui sont traités par de l'ammoniac ou une amine primaire pour conduire aux composés de formule (XVIII/a) et/ou (XVIII/b):

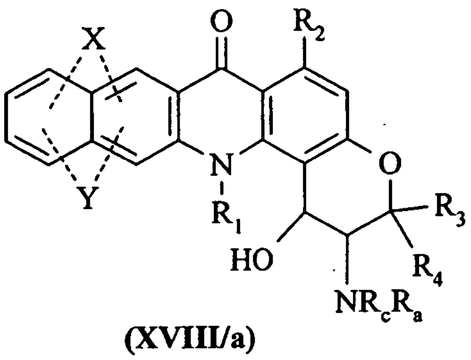

(XVIII/a)

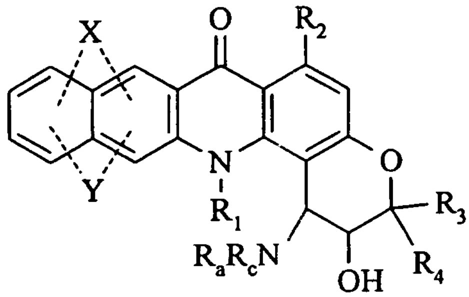

(XVIII/b)

dans lesquelles X, Y, $R_1$, $R_2$, $R_3$ et $R_4$, sont tels que définis précédemment, $R_c$ représente un atome d'hydrogène et $R_a$ représente un atome d'hydrogène dans le cas où le réactif utilisé est de l'ammoniac, ou un groupement choisi parmi alkyle ($C_1$-$C_6$) linéaire ou ramifié, aryle et arylalkyle ($C_1$-$C_6$) linéaire ou ramifié dans le cas où le réactif utilisé est une amine primaire,

composés de formules (XVIII/a) et (XVIII/b) dont la fonction alcool est protégée par un groupement protecteur des groupes hydroxy puis qui sont soumis à l'action d'un composé de formule (XVI) Hal-$G_1$ tel que défini précédemment, pour conduire respectivement aux composés de formules (XIX/a) et (XIX/b),

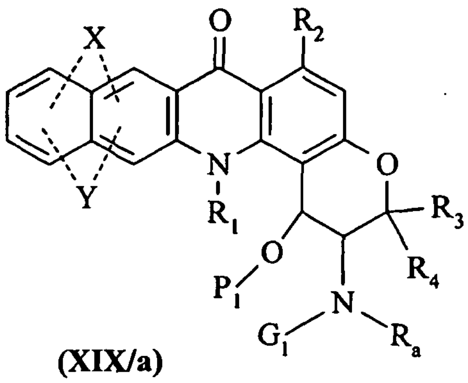

(XIX/a)

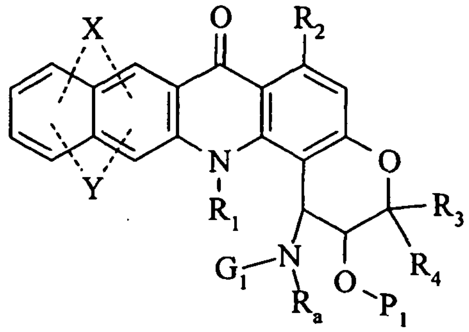

(XIX/b)

dans lesquelles X, Y, $R_1$, $R_2$, $R_3$, $R_4$, $R_a$, $G_1$ sont tels que définis précédemment, et $P_1$ représente un groupement protecteur des fonctions hydroxy,

composés de formules (XIX/a) et (XIX/b), dont on déprotège le groupement hydroxy pour conduire respectivement aux composés de formules (I/e1) et (I/f1), cas particuliers des composés de formule (I) :

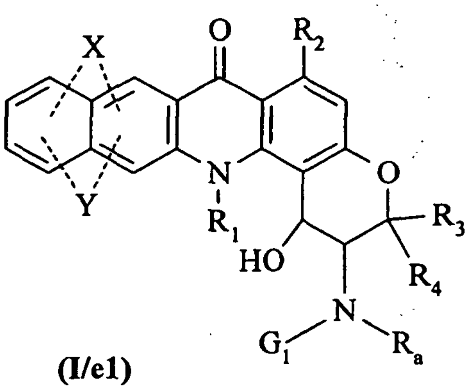

(I/e1)

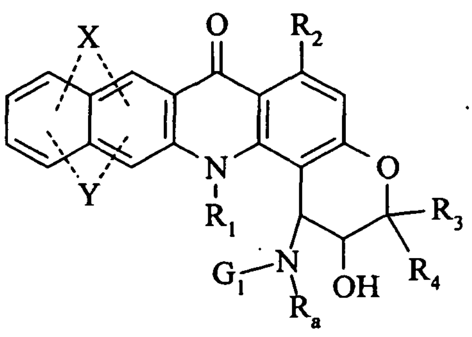

(I/f1)

dans lesquelles X, Y, $R_1$, $R_2$, $R_3$, $R_4$, $R_a$ et $G_1$ sont tels que définis précédemment, composés de formules (I/e1) et (1/f1) qui sont soumis :

❖ soit à l'action d'un anhydride de formule $(R_{30}CO)_2O$ dans laquelle $R_{30}$ représente un groupement alkyle ($C_1$-$C_6$) linéaire ou ramifié, aryle ou arylalkyle ($C_1$-$C_6$) linéaire ou ramifié, pour conduire respectivement aux composés de formule (I/e2) et (I/f2), cas particuliers des composés de formule (I) :

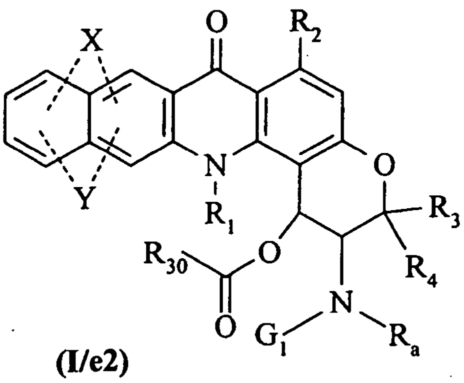

(I/e2)

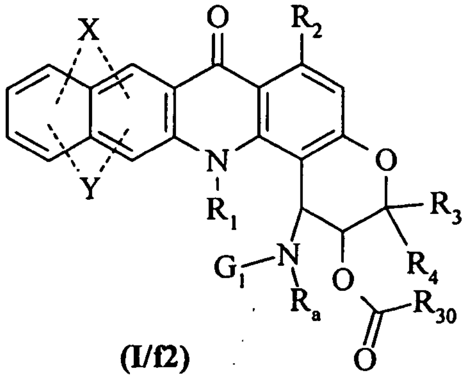

(I/f2)

dans lesquelles X, Y, $R_1$, $R_2$, $R_3$, $R_4$, $R_a$, $G_1$ et $R_{30}$ sont tels que définis précédemment,

❖ soit à des conditions de déshydratation en milieu acide, pour conduire respectivement aux composés de formule (I/e3) et (I/f3), cas particuliers des composés de formule (I) :

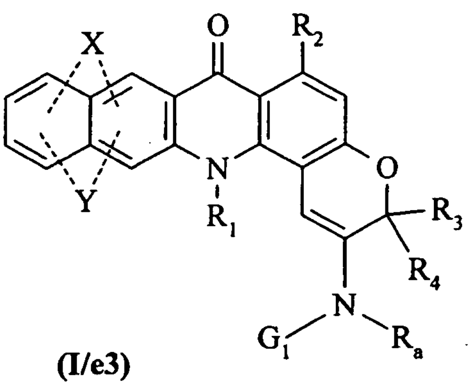

(I/e3)

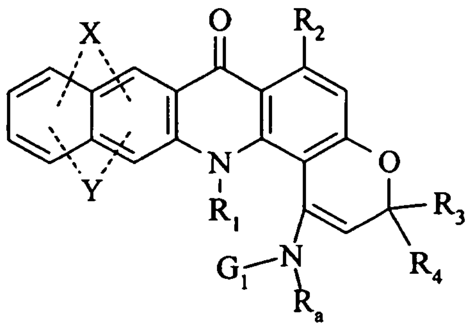

(I/f3)

dans lesquelles X, Y, $R_1$, $R_2$, $R_3$, $R_4$, $R_a$ et $G_1$ sont tels que définis précédemment,

c) ⇒ soit à l'action de $NaN_3$ en présence d'eau oxygénée, suivi d'une étape de réduction, pour conduire aux composés de formule (XX) :

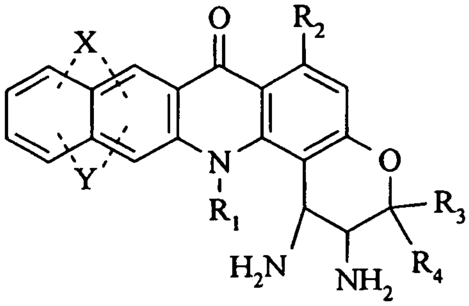

(XX)

dans laquelle X, Y, $R_1$, $R_2$, $R_3$ et $R_4$ sont tels que définis précédemment,
composés de formule (XX) qui sont soumis à l'action d'un composé de formule (XVI) Hal-$G_1$ tel que défini précédemment, pour conduire aux composés de formule (I/g), cas particulier des composés de formule (I) :

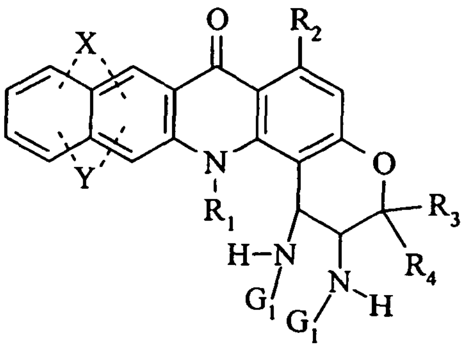

(I/g)

dans laquelle X, Y, $R_1$, $R_2$, $R_3$, $R_4$ et $G_1$ sont tels que définis précédemment,
d) ⇒ soit à l'action du permanganate de potassium en milieu polaire, pour conduire aux composés de formule (XXI) :

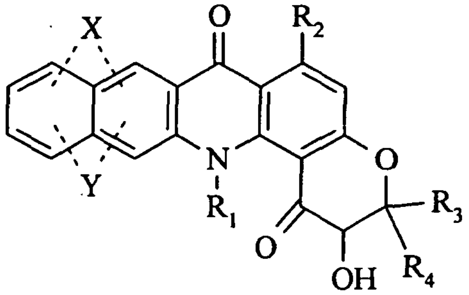

(XXI)

dans laquelle X, Y, $R_1$, $R_2$, $R_3$ et $R_4$ sont tels que définis précédemment,
composés de formule (XXI) dont on protège la fonction hydroxy par un groupement protecteur classiquement utilisé en synthèse organique, puis que l'on soumet à l'action de $P_2S_5$, pour conduire aux composés de formule (XXII) :

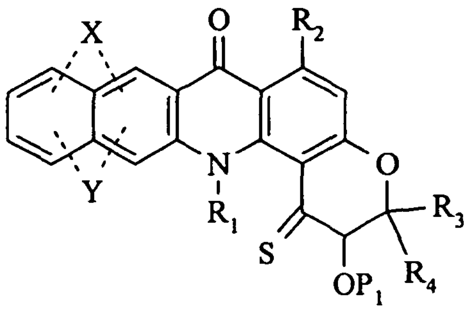

(XXII)

dans laquelle X, Y, $R_1$, $R_2$, $R_3$ et $R_4$ sont tels que définis précédemment, et $P_1$ représente un groupement protecteur des fonctions hydroxys,
composés de formule (XXII) qui est traité par un agent de réduction, puis soumis à une réaction de déprotection de la fonction hydroxy, pour conduire aux composés de formule (XXIII) :

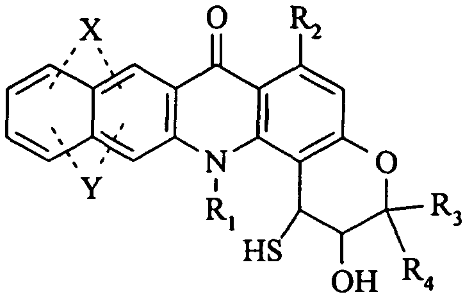

(XXIII)

dans laquelle X, Y, $R_1$, $R_2$, $R_3$ et $R_4$ sont tels que définis précédemment,
composés de formule (XXIII) qui est mis en présence d'un composé de formule (XVI) tel que défini précédemment, pour conduire aux composés de formule (I/i), cas particulier des composés de formule (I) :

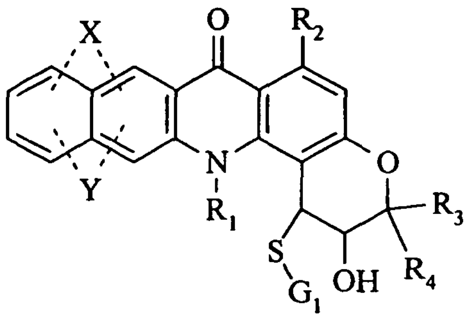

(I/i)

dans laquelle X, Y, $G_1$, $R_1$, $R_2$, $R_3$ et $R_4$ sont tels que définis précédemment,
composés de formule (I/i) qui peut être soumis à l'action d'un agent alkylant, d'un agent acylant, ou d'un composé de formule (XVIa) :

**Hal-G'$_1$** **(XVIa)**

dans laquelle Hal représente un atome d'halogène et G'$_1$ représente un groupement choisis parmi $-C(W_2)-W_3-T_1$, $-C(W_2)-T_1$, $-S(O)_n-W_3-T_1$ et $-S(O)_n-T_1$ dans lesquels $W_2$, $W_3$, $T_1$ et n sont tels que définis dans la formule (I), pour conduire aux composés de formule (I/j), cas particulier des composés de formule (I) :

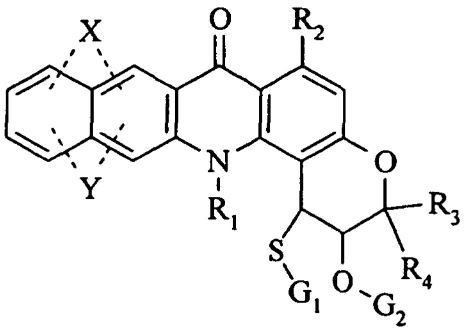

(I/j)

dans laquelle X, Y, $G_1$, $R_1$, $R_2$, $R_3$ et $R_4$ sont tels que définis précédemment et $G_2$ représente un groupement choisi parmi G'$_1$ tel que défini précédemment, alkoxy ($C_1$-$C_6$) linéaire ou ramifié, alkylcarbonyloxy ($C_1$-$C_6$) linéaire ou ramifié, arylcarbonyloxy, arylalkylcarbonyloxy ($C_1$-$C_6$) linéaire ou ramifié,
les composés (I/a) à (I/j), (I/$c_1$) à (I/$c_3$), (I/$d_1$) à (I/$d_3$), (I/$e_1$) à (I/$e_3$), (I/$f_1$) à (I/$f_3$), formant l'ensemble des composés de l'invention que l'on purifie, le cas échéant, selon une technique classique de purification, qui peuvent, si on le désire, être séparés en leurs différents isomères selon une technique classique de séparation et que l'on transforme, si on le souhaite, en leurs N-oxydes et, le cas échéant, en leurs sels d'addition à un acide ou à une base pharmaceutiquement acceptable.

**9.** Compositions pharmaceutiques contenant comme principe actif au moins un composé selon l'une quelconque des revendications 1 à 7, seul ou en combinaison avec un ou plusieurs excipients ou véhicules inertes non toxiques, pharmaceutiquement acceptables.

**10.** Compositions pharmaceutiques selon la revendication 9 contenant au moins un principe actif selon l'une quelconque des revendications 1 à 7 utiles, en tant que médicament, dans le traitement des cancers.

**11.** Compositions pharmaceutiques contenant comme principe actif au moins un composé de formule (XXI) telle que décrite dans la revendication 8, seule ou en combinaison avec un ou plusieurs excipients ou véhicules inertes non toxiques, pharmaceutiquement acceptable.

**12.** Composition pharmaceutique selon la revendication 11, contenant comme principe actif le 2-hydroxy-6-méthoxy-3,3-diméthyl-2,3-dihydro-1*H*-benzo[*b*]pyrano[3,2-*h*] acridine-1,7(14*H*)-dione seul ou en combinaison avec un ou plusieurs excipients ou véhicules inertes non toxiques, pharmaceutiquement acceptables

**13.** Composition pharmaceutique selon l'une quelconque des revendications 11 ou 12 utile en tant que médicament, dans le traitement des cancers.

## Claims

**1.** Compounds of formula (I) :

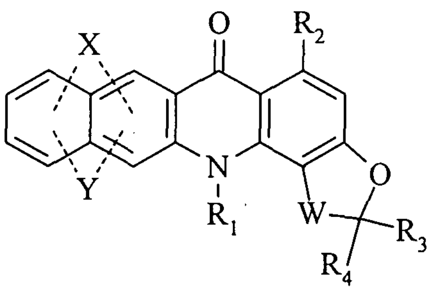

(I),

wherein :

• **X** and **Y**, which may be the same or different, each represents, independently of one another, a group selected from :

- hydrogen and halogen atoms,
- mercapto, cyano, nitro, linear or branched ($C_1$-$C_6$)alkyl, linear or branched ($C_1$-$C_6$)-trihaloalkyl and linear or branched trihalo-($C_1$-$C_6$)alkyl-carbonylamino groups,
- groups of formulae -ORa, -NRaRb, -NRa-C(O)-$T_1$, -O-C(O)-$T_1$, -O-$T_2$-NRaRb, -O-$T_2$-ORa, -NRa-$T_2$-NRaRb, -NRa-$T_2$-ORa and -NRa-$T_2$-$CO_2$Ra wherein :

  * **Ra** represents a group selected from a hydrogen atom and a linear or branched ($C_1$-$C_6$)alkyl group, an aryl group and an aryl-($C_1$-$C_6$)alkyl group wherein the alkyl moiety is linear or branched,
  * **Rb** represents a group selected from a hydrogen atom and a linear or branched ($C_1$-$C_6$)alkyl group, an aryl group and an aryl-($C_1$-$C_6$)alkyl group wherein the alkyl moiety is linear or branched,
  or
  **Ra+Rb,** together with the nitrogen atom carrying them, form a monocyclic 5- or 6-membered heterocycle optionally containing in the cyclic system a second hetero atom selected from oxygen and nitrogen,
  * **$T_1$** represents a group selected from linear or branched ($C_1$-$C_6$)alkyl, linear or branched ($C_2$-$C_6$) alkenyl, aryl, aryl-($C_1$-$C_6$)alkyl (wherein the alkyl moiety is linear or branched), and a linear or branched ($C_1$-$C_6$)alkylene chain substituted by .a group selected from -ORa and -NRaRb wherein Ra and Rb are as defined hereinbefore,
  * **$T_2$** represents a linear or branched ($C_1$-$C_6$)alkylene chain,

    - or X and Y, when they are in adjacent positions, together form a methylenedioxy group or an ethylenedioxy group,

it being understood that the substituents X and Y may be present on either of the two adjacent benzene rings,
• **$R_1$** represents a hydrogen atom or a linear or branched ($C_1$-$C_6$)alkyl group,
• **$R_2$** represents a group selected from a hydrogen atom and linear or branched ($C_1$-$C_6$)alkyl, -ORa, -NRaRb, -NRa-C(O)-$T_1$, -O-C(O)-$T_1$, -O-$T_2$-NRaRb, -O-$T_2$-ORa, -NRa-$T_2$-NRaRb, -NRa-$T_2$-ORa and -NRa-$T_2$-$CO_2$Ra groups, wherein Ra, Rb, $T_1$ and $T_2$ are as defined hereinbefore,
• **$R_3$, $R_4$**, which may be the same or different, represent, independently of one another, a hydrogen atom or a linear or branched ($C_1$-$C_6$)alkyl group,
• **W** represents a group of formula -CH($R_5$)-CH($R_6$)- wherein :

* **$R_5$ and/or $R_6$,** represent(s) a group selected from -$W_1$-C($W_2$)-$W_3$-$T_1$, -$W_1$-S(O)$_n$-$W_3$-$T_1$ and -$W_1$-S(O)$_n$-$T_1$ wherein :

  - $W_1$ represents an oxygen or sulphur atom or a nitrogen atom substituted by a hydrogen atom or by a linear or branched ($C_1$-$C_6$)alkyl group, an aryl group or an aryl-($C_1$-$C_6$)alkyl group wherein the alkyl moiety is linear or branched,
  - $W_2$ represents an oxygen atom or a sulphur atom,
  - $W_3$ represents an oxygen or sulphur atom or a nitrogen atom substituted by a hydrogen atom or by a linear or branched ($C_1$-$C_6$)alkyl group, an aryl group or an aryl-($C_1$-$C_6$)alkyl group wherein the alkyl moiety is linear or branched, a bond when $T_1$ represents a ($C_4$)alkenyl group,
  - $T_1$ is as defined hereinbefore,
  - n represents an integer selected from 1 and 2,

and in the case where only one of the two groups $R_5$ and $R_6$ represents a group as defined hereinbefore then the other of the said groups $R_5$ or $R_6$ represents a group selected from a hydrogen atom, hydroxy, linear or branched ($C_1$-$C_6$)alkoxy, linear or branched ($C_1$-$C_6$)alkyl-carbonyloxy, arylcarbonyloxy, aryl-($C_1$-$C_6$)alkyl-carbonyloxy (wherein the alkyl moiety is linear or branched), and amino optionally substituted by one or two, identical or different, linear or branched ($C_1$-$C_6$)alkyl groups,

their enantiomers, diastereoisomers and N-oxides, and addition salts thereof with a pharmaceutically acceptable acid or base.
aryl being understood to mean a phenyl or naphthyl group optionally containing one or more, identical or different, substituents selected from hydroxy, halogen, carboxy, nitro, amino, linear or branched ($C_1$-$C_6$)alkylamino, di($C_1$-$C_6$) alkylamino wherein each alkyl moiety may be linear or branched, linear or branched ($C_1$-$C_6$)alkoxy, linear or branched

$(C_1-C_6)$acyl and linear or branched $(C_1-C_6)$alkyl-carbonyloxy, and isomers being understood to comprise optical isomers.

**2.** Compounds of formula (I) according to claim 1, **characterised in that** $R_5$ and $R_6$ are identical and each represent a group of formula $-W_1-C(W_2)-W_3-T_1$ or $-W_1-S(O)_n-T_1$ wherein $W_1$, $W_2$, $W_3$, $T_1$ and n are as defined for formula (I), their enantiomers, diastereoisomers and N-oxides, and addition salts thereof with a pharmaceutically acceptable acid or base.

**3.** Compounds of formula (I) according to either claim 1 or claim 2, **characterised in that** $R_5$ and $R_6$ are identical and each represent a group of formula $-W_1-C(W_2)-W_3-T_1$ wherein $W_1$ represents an oxygen atom, $W_2$ represents an oxygen atom, $W_3$ represents a nitrogen atom substituted by a hydrogen atom or by a linear or branched $(C_1-C_6)$ alkyl group, an aryl group or an aryl-$(C_1-C_6)$alkyl group wherein the alkyl moiety is linear or branched and $T_1$ is as defined for formula (I), their enantiomers, diastereoisomers and N-oxides, and addition salts thereof with a pharmaceutically acceptable acid or base.

**4.** Compounds of formula (I) according to either claim 1 or claim 2, **characterised in that** $R_5$ and $R_6$ are identical and each represent a group of formula $-W_1-S(O)_n-T_1$ wherein $W_1$ represents an oxygen atom, $T_1$ is as defined for formula (I) and n is 2, their enantiomers, diastereoisomers and N-oxides, and addition salts thereof with a pharmaceutically acceptable acid or base.

**5.** Compounds of formula (I) according to any one of claims 1 to 4, **characterised in that** $R_3$ and $R_4$, which may be the same or different, represent a linear or branched $(C_1-C_6)$alkyl group, their enantiomers, diastereoisomers and N-oxides, and addition salts thereof with a pharmaceutically acceptable acid or base.

**6.** Compounds of formula (I) according to any one of claims 1 to 4, **characterised in that** $R_2$ represents a group selected from linear or branched $(C_1-C_6)$alkoxy, -NRaRb, $-O-T_2$-NRaRb, $-O-T_2$-ORa, -NRa-$T_2$-NRaRb and -NRa-$T_2$-ORa wherein Ra, Rb, $T_1$ and $T_2$ are as defined for formula (I), their enantiomers, diastereoisomers and N-oxides, and addition salts thereof with a pharmaceutically acceptable acid or base.

**7.** Compounds of formula (I) according to claim 1*, which are :

- (1S,2S)-1-{[(dimethylamino)carbonyl]oxy}-6-methoxy-3,3,14-trimethyl-7-oxo-2,3,7,14-tetrahydro-1*H*-benzo[*b*]pyrano[3,2-*h*]acridin-2-yl dimethylcarbamate
- and (1*S*,2*S*)-6-methoxy-3,3,14-trimethyl-2-{[(4-methylphenyl)sulphonyl]oxy}-7-oxo-2,3,7,14-tetrahydro-1*H*-benzo[*b*]pyrano[3,2-*h*]acridin-1-yl 4-methylbenzenesulphonate,

their enantiomers, diastereoisomers and N-oxides, and addition salts thereof with a pharmaceutically acceptable acid or base.

**8.** Process for the preparation of compounds of formula (I) according to claim 1, **characterised in that** there is used as starting material a compound of formula (VI) :

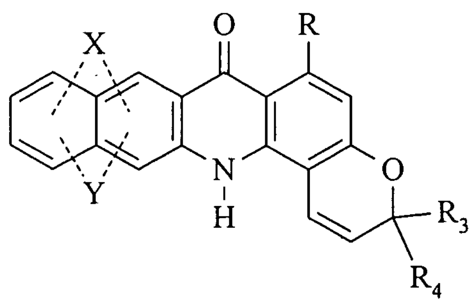

**(VI),**

wherein X, Y, R, $R_3$ and $R_4$ are as defined for formula (I),
the nitrogen atom of which compound of formula (VI) is optionally substituted, by the action of an alkyl halide or of a dialkyl sulphate in the presence of a deprotonating agent, in an aprotic polar solvent or under phase transfer conditions, to yield the compounds of formula (IX) :

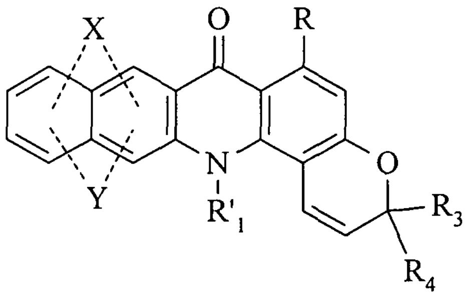

**(IX),**

wherein X, Y, R, $R_3$ and $R_4$ are as defined hereinbefore and $R'_1$ represents a linear or branched ($C_1$-$C_6$)alkyl group, which compounds of formula (IX) are subjected to the action of an alkylating agent or an acylating agent under customary conditions of organic synthesis to yield the compounds of formula (X) :

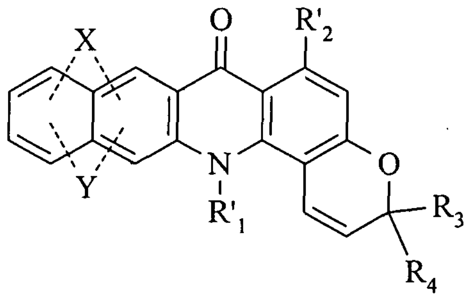

**(X),**

wherein X, Y, $R'_1$, $R_3$ and $R_4$ are as defined hereinbefore and $R'_2$ represents a group selected from -ORa, -O-C(O)-$T_1$, -O-$T_2$-NRaRb and -O-$T_2$-ORa wherein Ra, Rb, $T_1$ and $T_2$ are as defined for formula (I),
which compounds of formula (X), in the case where $R'_2$ represents an alkoxy group, are treated with a compound of formula (XI) :

$HNRaR_{10}$ **(XI),**

wherein Ra represents a hydrogen atom, a linear or branched ($C_1$-$C_6$)alkyl group, an aryl group or an aryl-($C_1$-$C_6$) alkyl group wherein the alkyl moiety is linear or branched, and $R_{10}$ represents a group selected from Rb, -C(O)-$T_1$, -$T_2$-NRaRb, -$T_2$-ORa and -$T_2$-$CO_2$Ra wherein Ra, Rb, $T_1$ and $T_2$ are as defined for formula (I),
to yield the compounds of formula (XII) :

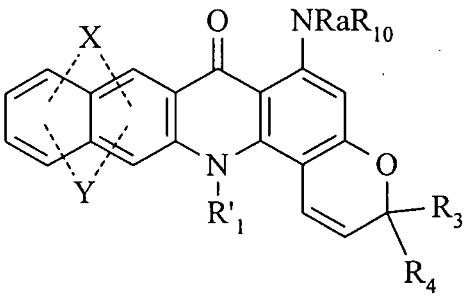

**(XII),**

wherein X, Y, $R'_1$, $R_3$, $R_4$, Ra and $R_{10}$ are as defined hereinbefore,
the totality of the compounds of formulae (VI), (IX), (X) and (XII) forming the compounds of formula (XIII) :

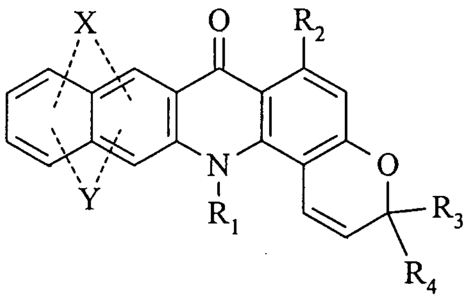

(XIII),

wherein X, Y, $R_1$, $R_2$, $R_3$ and $R_4$ are as defined in the general definition for formula (I), ***which compounds of formula (XIII) are subjected :***

a) ⇒ either to the action of an oxidising agent for alkene functions to yield the compounds of formula (XV) :

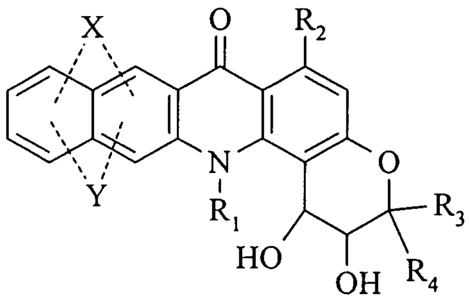

(XV),

wherein X, Y, $R_1$, $R_2$, $R_3$ and $R_4$ are as defined hereinbefore, which compounds of formula (XV) are treated with a compound of formula (XVI) :

**Hal-$G_1$** **(XVI),**

wherein Hal represents a halogen atom and $G_1$ represents a group selected from $-C(W_2)-W_3-T_1$, $-C(W_2)-T_1$, $-S(O)_n-W_3-T_1$ and $-S(O)_n-T_1$ wherein $W_2$, $W_3$, $T_1$ and n are as defined for formula (I), to yield the compounds of formulae (I/b), (I/c) and (I/d), particular cases of the compounds of formula (I) :

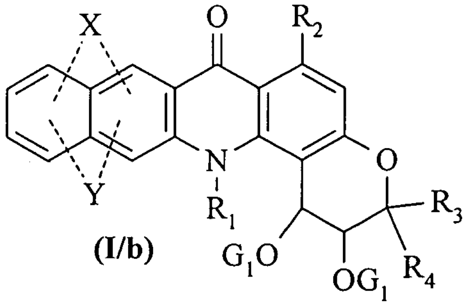

(I/b)

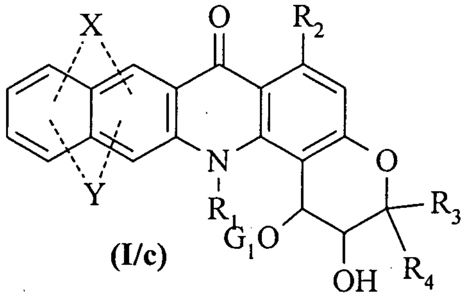

(I/c)

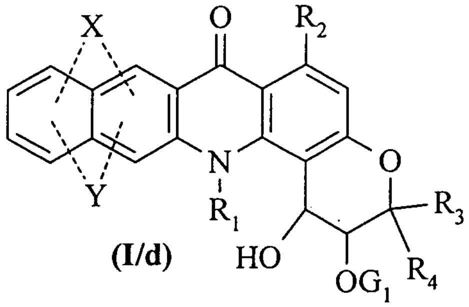

(I/d)

,

wherein X, Y, $R_1$, $R_2$, $R_3$, $R_4$ and $G_1$ are as defined hereinbefore, which compounds of formulae (I/c) and/or (I/d) are subjected :

- ❖ either to the action of an alcohol of formula $R_{20}$-OH, wherein $R_{20}$ represents a linear or branched ($C_1$-$C_6$) alkyl group, to yield the compounds of formulae (I/c1) and (1/d1), respectively :

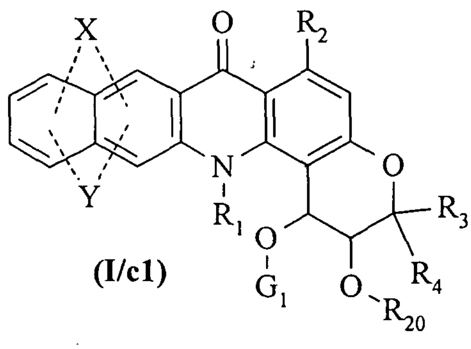

(I/c1)

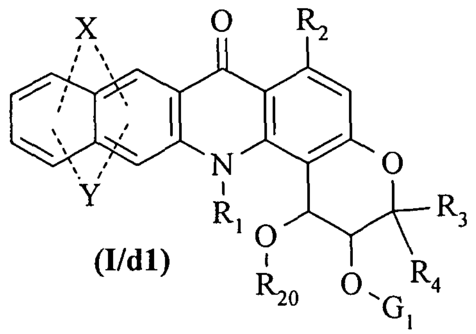

(I/d1)

,

wherein X, Y, $R_1$, $R_2$, $R_3$, $R_4$, $G_1$, and $R_{20}$ are as defined hereinbefore,

- ❖ or to the action of an anhydride of formula $(R_{30}CO)_2O$, wherein $R_{30}$ represents a linear or branched ($C_1$-$C_6$)alkyl group, an aryl group or an aryl-($C_1$-$C_6$)alkyl group wherein the alkyl moiety is linear or branched, to yield the compounds of formulae (I/c2) and (I/d2), respectively :

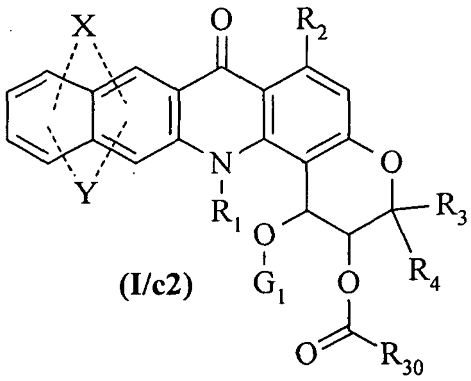
(I/c2)

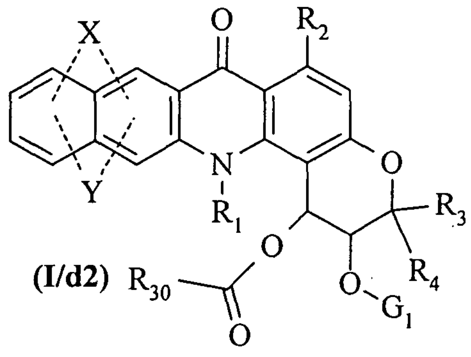
(I/d2)

,

wherein X, Y, $R_1$, $R_2$, $R_3$, $R_4$, $G_1$ and $R_{30}$ are as defined hereinbefore,

❖ or to dehydrating conditions in an acid medium to yield the compounds of formulae (1/c3) and (1/d3), respectively, particular cases of the compounds of formula (I) :

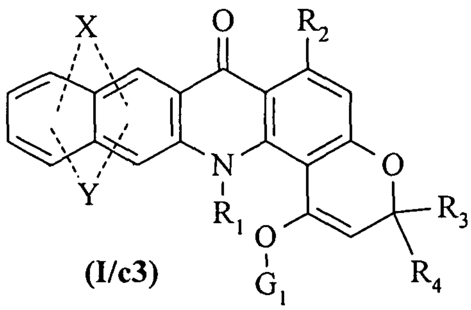
(I/c3)

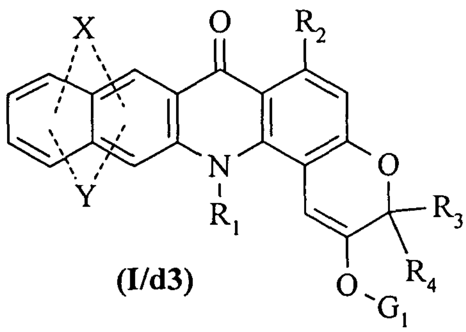
(I/d3)

,

wherein X, Y, $R_1$, $R_2$, $R_3$, $R_4$ and $G_1$ are as defined hereinbefore,

b) ⇒ or to the action of a peracid or of dimethyl dioxirane to yield the compounds of formula (XVII) :

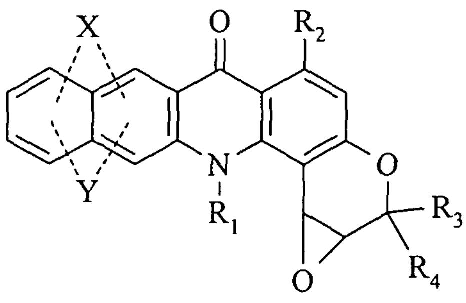
(XVII),

wherein X, Y, $R_1$, $R_2$, $R_3$ and $R_4$ are as defined hereinbefore,
which compounds of formula (XVII) are treated with ammonium hydroxide or with a primary amine to yield the compounds of formulae (XVIII/a) and/or (XVIII/b):

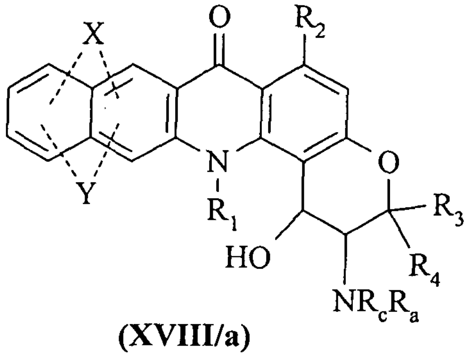
(XVIII/a)

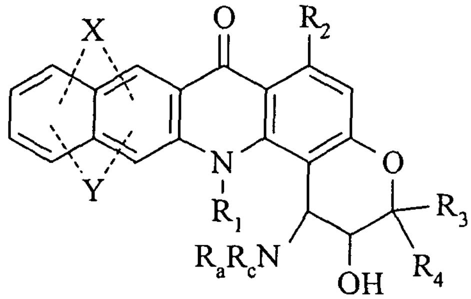
(XVIII/b)

,

wherein X, Y, $R_1$, $R_2$, $R_3$ and $R_4$ are as defined hereinbefore, $R_c$ represents a hydrogen atom and $R_a$ represents a hydrogen atom in the case where the reagent used is ammonium hydroxide or represents a group selected from linear or branched $(C_1\text{-}C_6)$alkyl, aryl and aryl-$(C_1\text{-}C_6)$alkyl (wherein the alkyl moiety is linear or branched) in the case where the reagent used is a primary amine,

the alcohol function of which compounds of formulae (XVIII/a) and (XVIII/b) is protected by a protecting group for hydroxy groups, the compounds then being subjected to the action of a compound of formula Hal-$G_1$ (XVI) as defined hereinbefore to yield the compounds of formulae (XIX/a) and (XIX/b), respectively,

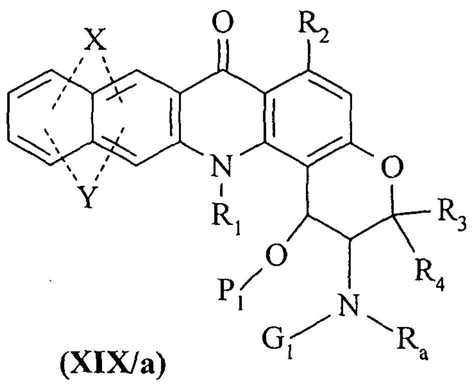
(XIX/a)

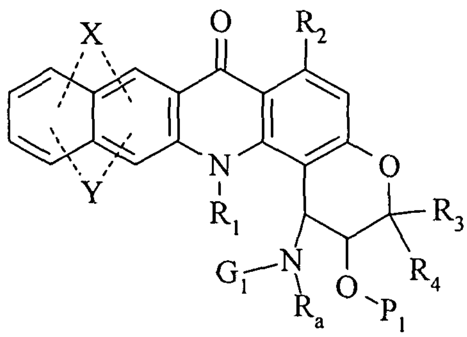
(XIX/b)

,

wherein X, Y, $R_1$, $R_2$, $R_3$, $R_4$, $R_a$ and $G_1$ are as defined hereinbefore and $P_1$ represents a protecting group for hydroxy functions,

the hydroxy group of which compounds of formulae (XIX/a) and (XIX/b) is deprotected to yield the compounds of formulae (1/e1) and (I/f1), respectively, particular cases of the compounds of formula (I) :

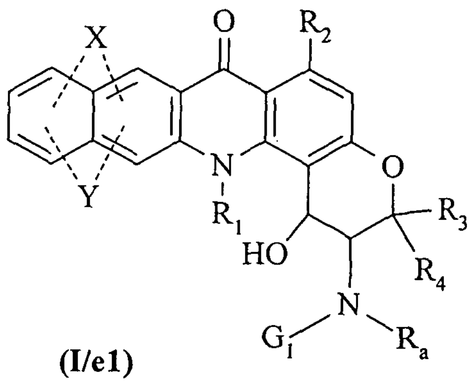
(I/e1)

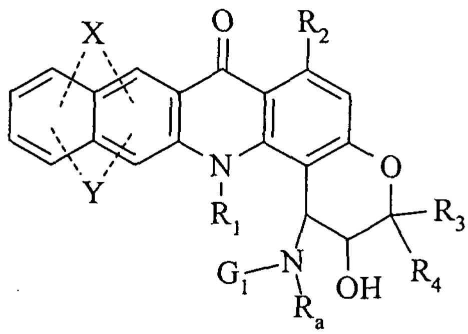
(I/f1)

,

wherein X, Y, $R_1$, $R_2$, $R_3$, $R_4$, $R_a$ and G, are as defined hereinbefore,

which compounds of formulae (I/e1) and (I/f1) are subjected :

❖ either to the action of an anhydride of formula $(R_{30}CO)_2O$, wherein $R_{30}$ represents a linear or branched $(C_1$-$C_6)$alkyl group, an aryl group or an aryl-$(C_1$-$C_6)$alkyl group wherein the alkyl moiety is linear or branched, to yield the compounds of formulae (I/e2) and (I/f2), respectively, particular cases of the compounds of formula (I) :

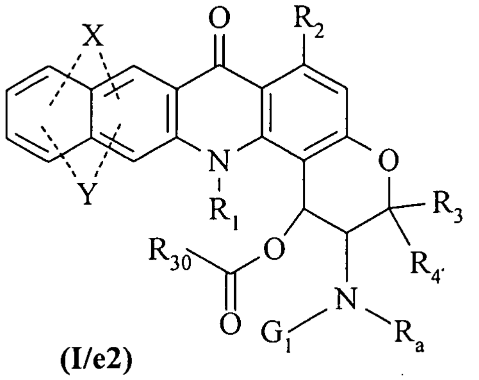
(I/e2)

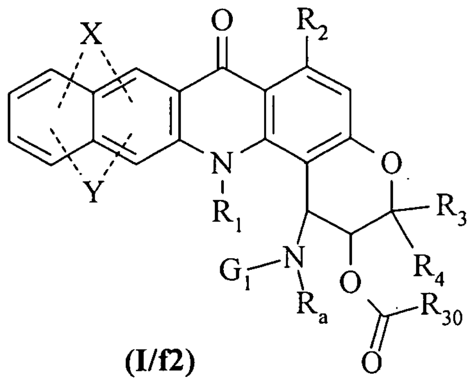
(I/f2) ,

wherein X, Y, $R_1$, $R_2$, $R_3$, $R_4$, $R_a$, $G_1$ and $R_{30}$ are as defined hereinbefore,

❖ or to dehydrating conditions in an acid medium to yield the compounds of formulae (I/e3) and (I/f3), respectively, particular cases of the compounds of formula (I) :

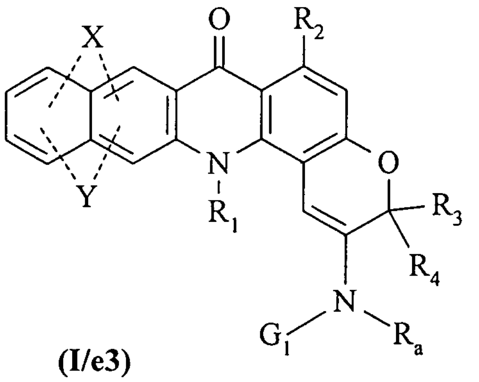
(I/e3)

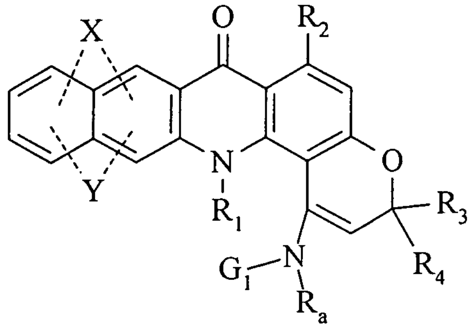
(I/f3) ,

wherein X, Y, $R_1$, $R_2$, $R_3$, $R_4$, $R_a$ and $G_1$ are as defined hereinbefore,

c) ⇒ or to the action of $NaN_3$ in the presence of hydrogen peroxide followed by a reduction step to yield the compounds of formula (XX):

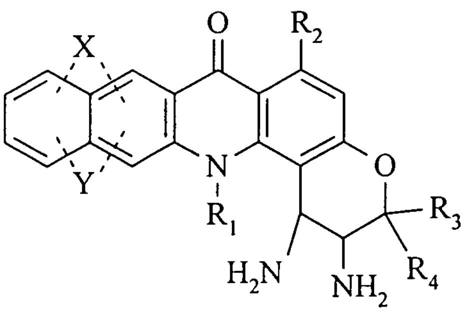
(XX),

wherein X, Y, $R_1$, $R_2$, $R_3$ and $R_4$ are as defined hereinbefore, which compounds of formula (XX) are subjected to the action of a compound of formula Hal-$G_1$ (XVI) as defined hereinbefore to yield the compounds of formula (I/g), a particular case of the compounds of formula (I) :

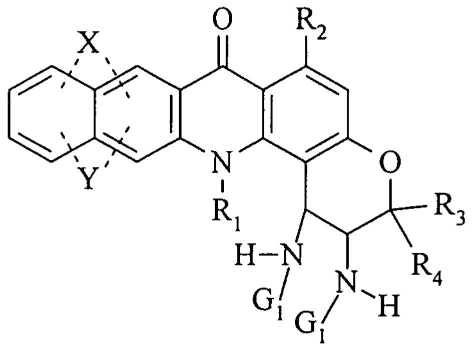

**(I/g),**

wherein X, Y, $R_1$, $R_2$, $R_3$, $R_4$ and $G_1$ are as defined hereinbefore,
d) ⇒ or to the action of potassium permanganate in a polar medium to yield the compounds of formula (XXI) :

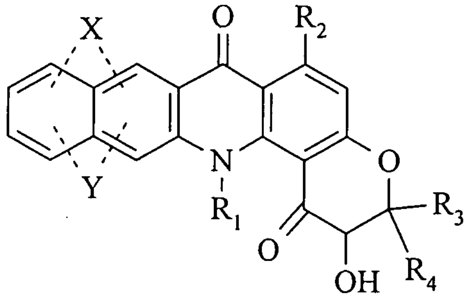

**(XXI),**

wherein X, Y, $R_1$, $R_2$, $R_3$ and $R_4$ are as defined hereinbefore, the hydroxy functions of which compounds of formula (XXI) are protected with a protecting group conventionally used in organic synthesis, the compounds then being subjected to the action of $P_2S_5$ to yield the compounds of formula (XXII) :

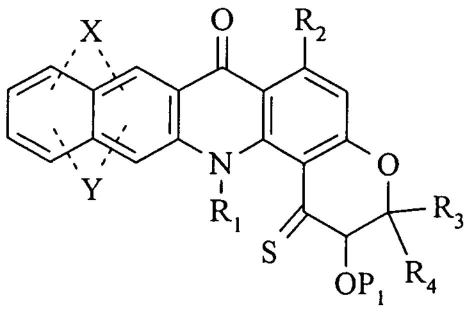

**(XXII),**

wherein X, Y, $R_1$, $R_2$, $R_3$ and $R_4$ are as defined hereinbefore and $P_1$ represents a protecting group for hydroxy functions,
which compounds of formula (XXII) are treated with a reducing agent and then subjected to a reaction for deprotection of the hydroxy function to yield the compounds of formula (XXIII) :

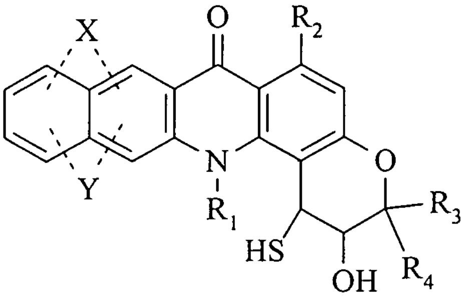

(XXIII),

wherein X, Y, $R_1$, $R_2$, $R_3$ and $R_4$ are as defined hereinbefore, which compounds of formula (XXIII) are treated with a compound of formula (XVI) as defined hereinbefore to yield the compounds of formula (I/i), a particular case of the compounds of formula (I) :

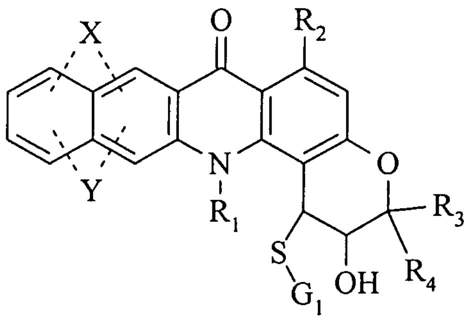

(I/i),

wherein X, Y, $G_1$, $R_1$, $R_2$, $R_3$ and $R_4$ are as defined hereinbefore, which compounds of formula (I/i) may be subjected to the action of an alkylating agent, an acylating agent or a compound of formula (XVIa) :

**Hal-G'$_1$ (XVIa),**

wherein Hal represents a halogen atom and G'$_1$ represents a group selected from $-C(W_2)-W_3-T_1$, $-C(W_2)-T_1$, $-S(O)_n-W_3-T_1$ and $-S(O)_n-T_1$, wherein $W_2$, $W_3$, $T_1$ and n are as defined for formula (I), to yield the compounds of formula (I/j), a particular case of the compounds of formula (I) :

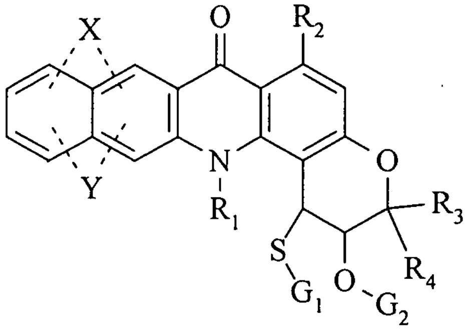

(I/j),

wherein X, Y, $G_1$, $R_1$, $R_2$, $R_3$ and $R_4$ are as defined hereinbefore and $G_2$ represents a group selected from G'$_1$ as defined hereinbefore, linear or branched $(C_1-C_6)$alkoxy, linear or branched $(C_1-C_6)$alkyl-carbonyloxy, aryl-carbonyloxy and aryl-$(C_1-C_6)$alkyl-carbonyloxy (wherein the alkyl moiety is linear or branched), the compounds (I/a) to (I/j), (I/$c_1$) to (I/$c_3$), (I/$d_1$) to (I/$d_3$), (I/$e_1$) to (I/$e_3$) and (I/$f_1$) to (I/$f_3$) constituting the totality of the compounds of the invention, which are purified, if necessary, according to a conventional purification technique, which may be, if desired, separated into their different isomers according to a conventional separation

technique and which are converted, if desired, into their N-oxides and, where appropriate, their addition salts with a pharmaceutically acceptable acid or base.

**9.** Pharmaceutical compositions comprising as active ingredient at least one compound according to any one of claims 1 to 7, alone or in combination with one or more inert, non-toxic, pharmaceutically acceptable excipients or carriers.

**10.** Pharmaceutical compositions according to claim 9 comprising at least one active ingredient according to any one of claims 1 to 7 for use as a medicament in the treatment of cancers.

**11.** Pharmaceutical compositions comprising as active ingredient at least one compound of formula (XXI) as described in claim 8, alone or in combination with one or more inert, non-toxic, pharmaceutically acceptable excipients or carriers.

**12.** Pharmaceutical composition according to claim 11 comprising as active ingredient 2-hydroxy-6-methoxy-3,3-dimethyl-2,3-dihydro-1*H*-benzo[*b*]pyrano[3,2-*h*]acridine-1,7(14*H*)-dione, alone or in combination with one or more inert, non-toxic, pharmaceutically acceptable excipients or carriers

**13.** Pharmaceutical composition according to either claim 11 or claim 12 for use as a medicament in the treatment of cancers.

**Patentansprüche**

**1.** Verbindungen der Formel (I):

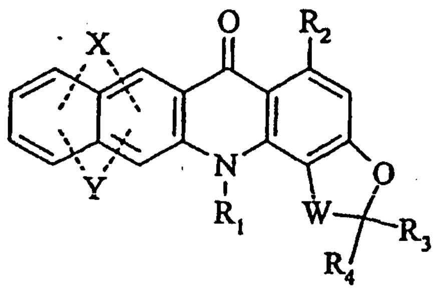

**(I)**

in der:

• **X** und **Y**, die gleichartig oder verschieden sind, unabhängig voneinander jeweils eine Gruppe bedeuten ausgewählt aus:

- Wasserstoff- und Halogenatomen,
- Mercapto-, Cyano-, Nitro-, geradkettige oder verzweigte ($C_1$-$C_6$)-Alkyl-, geradkettige oder verzweigte ($C_1$-$C_6$)-Trihalogenalkyl- und geradkettige oder verzweigte ($C_1$-$C_6$)-Trihalogenalkylcarbonylamino-gruppen,
- Gruppen der Formeln ORa, NRaRb, -NRa-C(O)-$T_1$, -O-C(O)-$T_1$, -O-$T_2$-NRaRb, -O-$T_2$-ORa, -NRa-$T_2$-NRaRb, -NRa-$T_2$-ORa und -NRa-$T_2$-$CO_2$Ra, in denen:

* **Ra** eine Gruppe darstellt ausgewählt aus Wasserstoffatomen, geradkettigen oder verzweigten ($C_1$-$C_6$)-Alkylgruppen und geradkettigen oder verzweigten Aryl-($C_1$-$C_6$)-alkylgruppen,
* **Rb** eine Gruppe darstellt ausgewählt aus Wasserstoffatomen, geradkettigen oder verzweigten ($C_1$-$C_6$)-Alkylgruppen und geradkettigen oder verzweigten Aryl-($C_1$-$C_6$)-alkylgruppen.
oder
**Ra + Rb** gemeinsam mit dem sie tragenden Stickstoffatom einen monocyclischen Heterocyclus mit 5 bis 6 Kettengliedern bilden, der gegebenenfalls im Rahmen des cyclischen Systems ein zweites Heteroatom ausgewählt aus Sauerstoff und Stickstoff enthält,
* **$T_1$,** eine Gruppe darstellt ausgewählt aus geradkettigem oder verzweigtem (C,-$C_6$)-Alkyl, geradkettigem oder verzweigtem ($C_2$-$C_6$)-Alkenyl, Aryl, geradkettigem oder verzweigtem Aryl-($C_1$-$C_6$)-alkyl und

einer geradkettigen oder verzweigten ($C_1$-$C_6$)-Alkylenkette, die durch eine Gruppe substituiert ist ausgewählt aus -ORa und -NRaRb, worin Ra und Rb die oben angegebenen Bedeutungen besitzen, und
* **$T_2$** eine geradkettige oder verzweigte ($C_1$-$C_6$)-Alkylenkette darstellt,

- oder X und Y, wenn sie in benachbarten Positionen stehen, gemeinsam eine Methylendioxygruppe oder Ethylendioxygruppe bedeuten,

mit der Maßgabe, daß die Substituenten X und Y an dem einen oder dem anderen der beiden benachbarten Benzolringe vorliegen können,
• **$R_1$** ein Wasserstoffatom oder eine geradkettige oder verzweigte ($C_1$-$C_6$)-Alkylgruppe bedeutet,
• **$R_2$** eine Gruppe bedeutet ausgewählt aus Wasserstoffatomen, geradkettigen oder verzweigten ($C_1$-$C_6$)-Alkylgruppen und Gruppen ORa, NRaRb, -NRa-C(O)-$T_1$, -O-C(O)-$T_1$, -O-$T_2$-NRaRb, -O-$T_2$-ORa, -NRa-$T_2$-NRaRb, -NRa-$T_2$-ORa und -NRa-$T_2$-$CO_2$Ra, worin Ra, Rb, $T_1$ und $T_2$ die oben angegebenen Bedeutungen besitzen,
• **$R_3$** und **$R_4$,** die gleichartig oder verschieden sind, unabhängig voneinander ein Wasserstoffatom oder eine geradkettige oder verzweigte ($C_1$-$C_6$)-Alkylgruppe bedeuten,
• **W** eine Gruppe der Formel -CH($R_5$)-CH($R_6$)- bedeutet, in der:

• **$R_5$ und/oder $R_6$** eine Gruppe darstellen ausgewählt aus -$W_1$-C($W_2$)-$W_3$-$T_1$, -$W_1$-S(O)$_n$-$W_3$-$T_1$ und -$W_1$-S(O)$_n$-$T_1$, in denen:

- $W_1$ ein Sauerstoffatom, ein Schwefelatom oder ein durch ein Wasserstoffatom substituiertes Stickstoffatom, eine geradkettige oder verzweigte ($C_1$-$C_6$)-Alkylgruppe, Arylgruppe oder geradkettige oder verzweigte Aryl-($C_1$-$C_6$)-alkylgruppe darstellt,
- $W_2$ ein Sauerstoffatom oder ein Schwefelatom darstellt,
- $W_3$ ein Sauerstoffatom, ein Schwefelatom oder ein durch ein Wasserstoffatom substituiertes Stickstoffatom, eine geradkettige oder verzweigte ($C_1$-$C_6$)-Alkylgruppe, Arylgruppe oder geradkettige oder verzweigte Aryl-($C_1$-$C_6$)-alkylgruppe darstellt oder eine Bindung, wenn $T_1$ eine $C_4$-Alkenylgruppe bedeutet,
- $T_1$ die oben angegebenen Bedeutungen besitzt und
- n eine ganze Zahl ausgewählt aus 1 oder 2 darstellt,

wobei dann, wenn eine der beiden Gruppen $R_5$ und $R_6$ eine Gruppe der oben definierten Art darstellt, die andere der genannten Gruppen $R_5$ oder $R_6$ eine Gruppe bedeutet ausgewählt aus dem Wasserstoffatom, Hydroxy, geradkettigem oder verzweigtem ($C_1$-$C_6$)-Alkoxy, geradkettigem oder verzweigtem ($C_1$-$C_6$)-Alkylcarbonyloxy, Arylcarbonyloxy, geradkettigem oder verzweigtem Aryl-($C_1$-$C_6$)-alkylcarbonyloxy und gegebenenfalls durch eine oder zwei gleichartige oder verschiedenartige, geradkettige oder verzweigte ($C_1$-$C_6$)-Alkylgruppen substituiertem Amino, deren Enantiomere, Diastereoisomere, N-Oxide sowie deren Additionssalze mit einer pharmazeutisch annehmbaren Säure oder Base,
mit der Maßgabe, daß man unter Aryl eine Phenyl- oder Naphthylgruppe versteht, die gegebenenfalls einen oder mehrere gleichartige oder verschiedenartige Substituenten aufweist, ausgewählt aus Hydroxy, Halogen, Carboxy, Nitro, Amino, Alkylamino oder geradkettigem oder verzweigtem ($C_1$-$C_6$)-Dialkylamino, geradkettigem oder verzweigtem ($C_1$-$C_6$)-Alkoxy, geradkettigem oder verzweigtem ($C_1$-$C_6$)-Acyl und geradkettigem oder verzweigtem ($C_1$-$C_6$)-Alkylcarbonyloxy, und man unter Isomeren die optischen Isomeren versteht.

2. Verbindungen der Formel (I) nach Anspruch 1, **dadurch gekennzeichnet, dass** $R_5$ und $R_6$, die identisch sind und eine Gruppe der Formel -$W_1$-C($W_2$)-$W_3$-$T_1$ oder -$W_1$-S(O)$_n$-$T_1$ darstellen, in der $W_1$, $W_2$, $W_3$, $T_1$ und n die bezüglich der Formel (I) angegebenen Bedeutungen besitzen, deren Enantiomere, Diastereoisomere, N-Oxide sowie deren Additionssalze mit einer pharmazeutisch annehmbaren Säure oder Base.

3. Verbindungen der Formel (I) nach einem der Ansprüche 1 bis 2, **dadurch gekennzeichnet, dass** $R_5$ und $R_6$, die identisch sind und eine Gruppe der Formel -$W_1$-C($W_2$)-$W_3$-$T_1$ darstellen, in der $W_1$ ein Sauerstoffatom, $W_2$ ein Sauerstoffatom, $W_3$ ein Stickstoffatom, welches durch ein Wasserstoffatom, eine geradkettige oder verzweigte ($C_1$-$C_6$)-Alkylgruppe, Arylgruppe oder geradkettige oder verzweigte Aryl-($C_1$-$C_6$)-alkylgruppe substituiert ist, bedeuten und $T_1$ die bezüglich der Formel (I) angegebenen Bedeutungen besitzet, deren Enantiomere, Diastereoisomere, N-Oxide sowie deren Additionssalze mit einer pharmazeutisch annehmbaren Säure oder Base.

4. Verbindungen der Formel (I) nach einem der Ansprüche 1 bis 2, **dadurch gekennzeichnet, dass** $R_5$ und $R_6$, die

identisch sind und eine Gruppe der Formel $-W_1-S(O)_n-T_1$ bedeuten, in der $W_1$ ein Sauerstoffatom darstellt, $T_1$ die bezüglich der Formel (I) angegebenen Bedeutungen besitzt und n den Wert 2 besitzt, deren Enantiomere, Diastereoisomere, N-Oxide sowie deren Additionssalze mit einer pharmazeutisch annehmbaren Säure oder Base.

**5.** Verbindungen der Formel (I) nach einem der Ansprüche 1 bis 4, **dadurch gekennzeichnet, dass** $R_3$ und $R_4$, die gleichartig oder verschieden sind, eine geradkettige oder verzweigte $(C_1-C_6)$-Alkylgruppe bedeuten, deren Enantiomere, Diastereoisomere, N-Oxide sowie deren Additionssalze mit einer pharmazeutisch annehmbaren Säure oder Base.

**6.** Verbindungen der Formel (I) nach einem der Ansprüche 1 bis 4, **dadurch gekennzeichnet, dass** $R_2$ eine Gruppe bedeutet ausgewählt aus geradkettigem oder verzweigtem $(C_1-C_6)$-Alkoxy, NRaRb, $-O-T_2$-NRaRb, $-O-T_2$-ORa, -NRa-$T_2$-NRaRb und -NRa-$T_2$-ORa, in denen Ra, Rb, $T_1$ und $R_2$ die bezüglich der Formel (I) angegebenen Bedeutungen besitzen, deren Enantiomere, Diastereoisomere, N-Oxide sowie deren Additionssalze mit einer pharmazeutisch annehmbaren Säure oder Base.

**7.** Verbindungen der Formel (I) nach Anspruch 1, nämlich:

- (1*S*,2*S*)-1-{[(Dimethylamino)-carbonyl]-oxy}-6-methoxy-3,3,14-trimethyl-7-oxo-2,3,7,14-tetrahydro-1*H*-benzo[*b*]pyrano[3,2-*h*]acridin-2-yl-dimethylcarbamat und
- (1*S*,2*S*)-6-Methoxy-3,3,14-trimethyl-2-{[(4-methylphenyl)-sulfonyl]-oxy}-7-oxo-2,3,7,14-tetrahydro-1*H*-benzo[*b*]pyrano[3,2-*h*]acridin-1-yl-4-methylbenzolsulfonat,

deren Enantiomere, Diastereoisomere, N-Oxide sowie deren Additionssalze mit einer pharmazeutisch annehmbaren Säure oder Base.

**8.** Verfahren zur Herstellung der Verbindungen der Formel (I) nach Anspruch 1, **dadurch gekennzeichnet, dass** man als Ausgangsprodukt eine Verbindung der Formel (VI) verwendet:

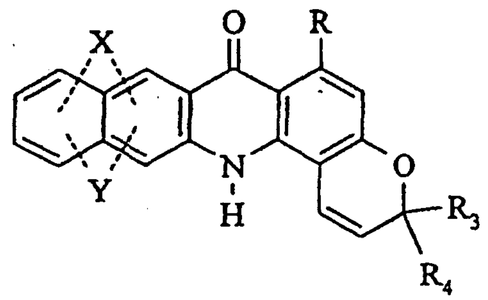

(VI)

in der X, Y, R, $R_3$ und $R_4$ die bezüglich der Formel (I) angegebenen Bedeutungen besitzen,
von welcher Verbindung der Formel (VI) man gegebenenfalls das Stickstoffatom durch Einwirkung eines Alkylhalogenids oder eines Dialkylsulfats in Gegenwart eines Deprotonierungsmittels in einem aprotischen polaren Lösungsmittel oder unter Bedingungen des Phasentransfers substituiert zur Bildung der Verbindungen der Formel (IX):

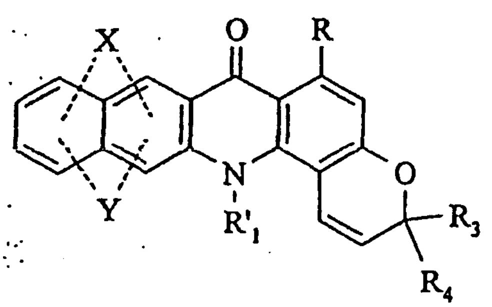

(IX)

in der X, Y, R, $R_3$ und $R_4$ die oben angegebenen Bedeutungen besitzen und $R'_1$ eine geradkettige oder verzweigte

($C_1$-$C_6$)-Alkylgruppe bedeutet,
welche Verbindungen der Formel (IX) man der Einwirkung eines Alkylierungsmittels oder eines Acylierungsmittels bei klassischen Bedingungen der organischen Synthese unterwirft zur Bildung der Verbindung der Formel (X):

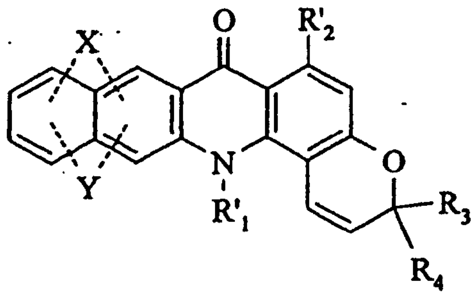

**(X)**

in der X, Y, $R'_1$, $R_3$ und $R_4$ die oben angegebenen Bedeutungen besitzen und $R'_2$ eine Gruppe bedeutet ausgewählt aus ORa, -O-C(O)-$T_1$, -O-$T_2$-NRaRb, -O-$T_2$-ORa, worin Ra, Rb, $T_1$ und $T_2$ die bezüglich der Formel (I) angegebenen Bedeutungen besitzen,
welche Verbindungen der Formel (X) man dann, wenn $R'_2$ eine Alkoxygruppe bedeutet, mit einer Verbindung der Formel (XI) behandelt:

$HNRaR_{10}$ **(XI)**

in der Ra ein Wasserstoffatom, eine geradkettige oder verzweigte ($C_1$-$C_6$)-Alkylgruppe, Arylgruppe oder geradkettige oder verzweigte Aryl-($C_1$-$C_6$)-alkylgruppe bedeutet und $R_{10}$ eine Gruppe bedeutet ausgewählt aus Rb, -C(O)-$T_1$, -$T_2$-NRaRb, -$T_2$-ORa und -$T_2$-$CO_2$Ra, in denen Ra, Rb, $T_1$ und $T_2$ die bezüglich der Formel (I) angegebenen Bedeutungen besitzen,
zur Bildung der Verbindungen der Formel (XII):

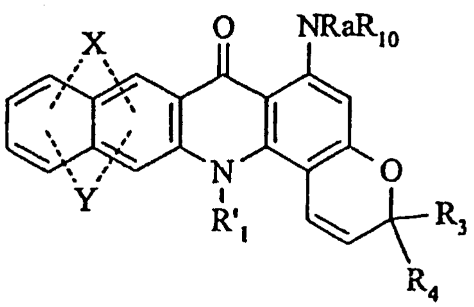

**(XII)**

in der X, Y, $R'_1$, $R_3$, $R_4$ Ra und $R_{10}$ die oben angegebenen Bedeutungen besitzen, wobei die Gesamtheit der Verbindungen der Formeln (VI), (IX), (X) und (XII) die Verbindungen der Formel (XIII) bildet:

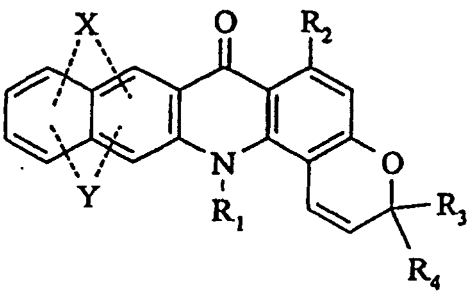

**(XIII)**

in der X, Y, $R_1$, $R_2$, $R_3$ und $R_4$ die bezüglich der allgemeinen Formel (I) angegebenen Bedeutungen besitzen, ***welche Verbindungen der Formel (XIII) man:***

a) ⇒ entweder der Einwirkung eines Mittels zur Oxidation der Alkenfunktionen unterwirft zur Bildung der Verbindungen der Formel (XV):

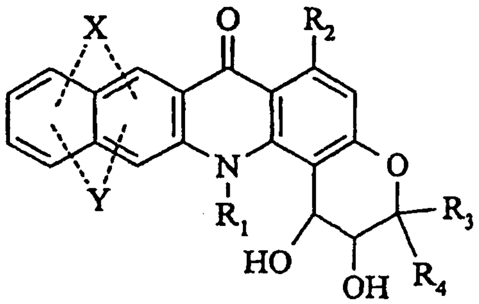

**(XV)**

in der X, Y, $R_1$, $R_2$, $R_3$ und $R_4$ die oben angegebenen Bedeutungen besitzen, welche Verbindungen der Formel (XV) man mit einer Verbindung der Formel (XVI) behandelt:

**Hal - $G_1$ (XVI)**

in der Hal ein Halogenatom darstellt und $G_1$ eine Gruppe bedeutet ausgewählt aus $-C(W_2)-W_3-T_1$, $-C(W_2)-T_1$, $-S(O)_n-W_3-T_1$ und $-S(O)_n-T_1$, worin $W_2$, $W_3$, $T_1$ und n die bezüglich der Formel (I) angegebenen Bedeutungen besitzen,
zur Bildung der Verbindungen der Formeln (I/b), (I/c) und (I/d), Sonderfällen der Verbindungen der Formel (I):

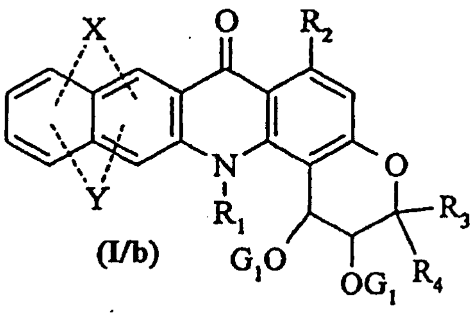

**(I/b)**

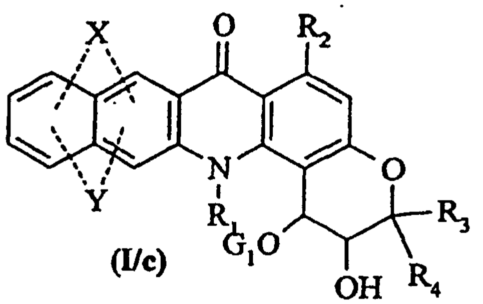

**(I/c)**

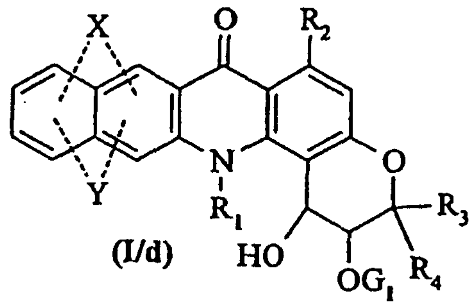

**(I/d)**

in denen X, Y, $R_1$, $R_2$, $R_3$, $R_4$ und $G_1$ die oben angegebenen Bedeutungen besitzen, welche Verbindungen der Formeln (I/c) und/oder (I/d) man:

❖ entweder der Einwirkung eines Alkohols der Formel $R_{20}$-OH, worin $R_2$ eine geradkettige oder verzweigte ($C_1$-$C_6$)-Alkylgruppe bedeutet, unterwirft zur Bildung der Verbindungen der Formeln (I/c1) bzw. (I/d1):

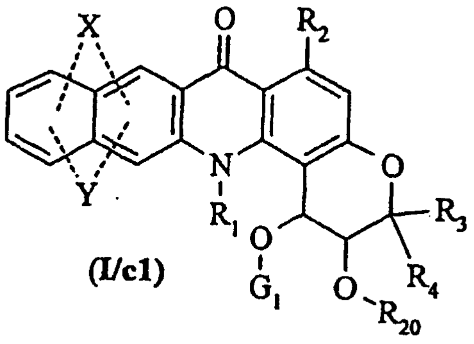

(I/c1)

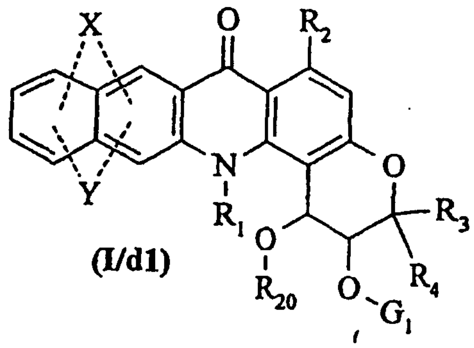

(I/d1)

in denen X, Y, $R_1$, $R_{20}$, $R_3$, $R_4$, $G_1$ und $R_{20}$ die oben angegebenen Bedeutungen besitzen,

❖ oder der Einwirkung eines Anhydrids der Formel $(R_{30}CO)_2O$, in der $R_{30}$ eine geradkettige oder verzweigte ($C_1$-$C_6$)-Alkylgruppe, Arylgruppe oder geradkettige oder verzweigte Aryl-($C_1$-$C_6$)-alkylgruppe bedeutet, unterwirft zur Bildung der Verbindungen der Formeln (I/c2) bzw. (I/d2):

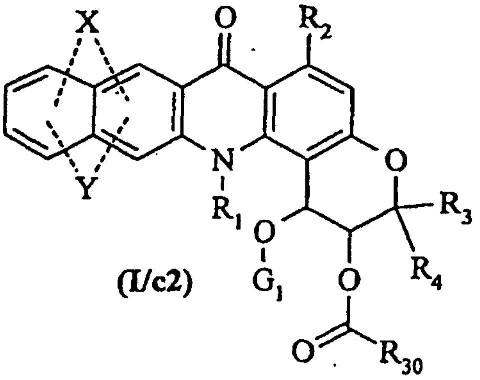

(I/c2)

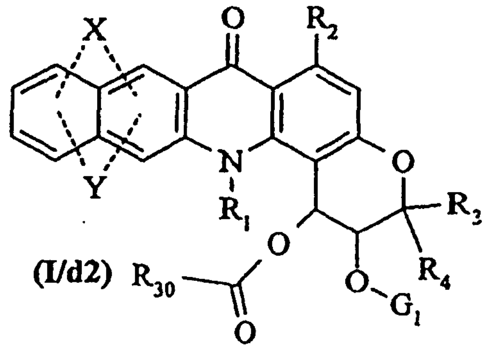

(I/d2)

in denen X, Y, $R_1$, $R_2$, $R_3$, $R_4$, $G_1$ und $R_{30}$ die oben angegebenen Bedeutungen besitzen,

❖ oder den Bedingungen der Dehydratisierung in saurem Medium unterwirft zur Bildung der Verbindungen der Formeln (I/c3) bzw. (I/d3), Sonderfällen der Verbindungen der Formel ((I):

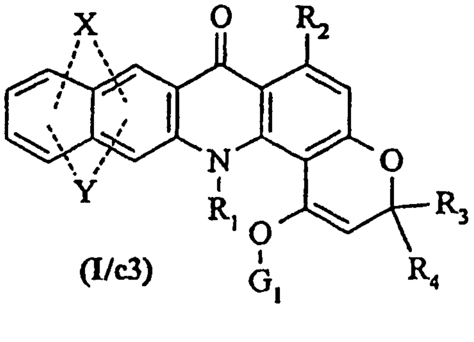

(I/c3)

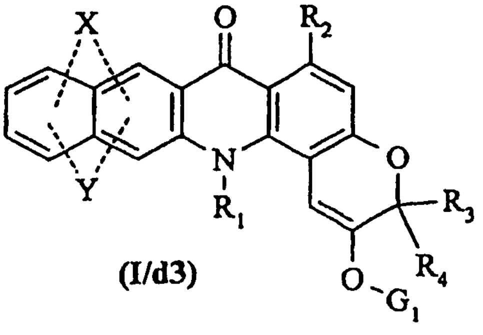

(I/d3)

in denen X, Y, $R_1$, $R_2$, $R_3$, $R_4$ und $G_1$ die oben angegebenen Bedeutungen besitzen,

b) ⇒ oder der Einwirkung einer Persäure oder von Dimethyldioxiran unterwirft zur Bildung der Verbindungen der Formel (XVII):

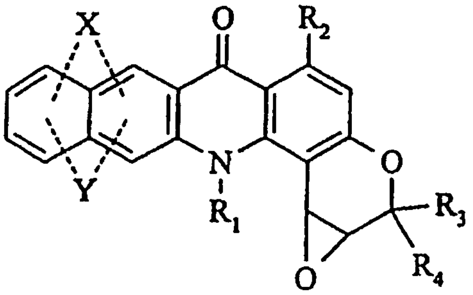

(XVII)

in der X, Y, $R_1$, $R_2$, $R_3$ und $R_4$ die oben angegebenen Bedeutungen besitzen, welche Verbindungen der Formel (XVII) man mit Ammoniak oder mit einem primären Amin behandelt zur Bildung der Verbindungen der Formeln (XVIII/a) und/ oder (XVIII/b):

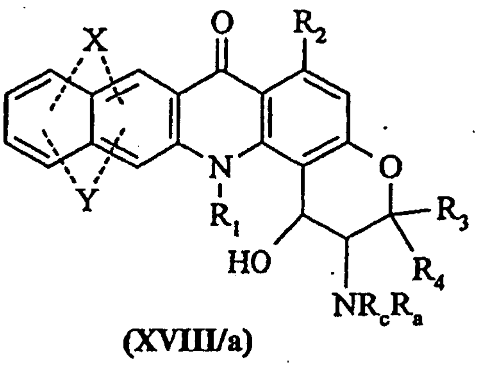

(XVIII/a)

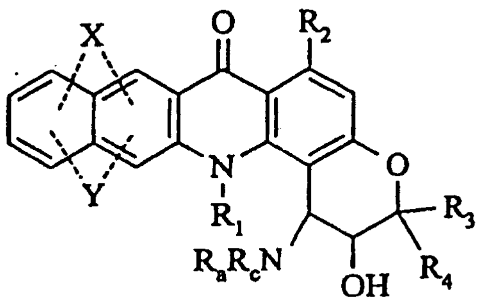

(XVIII/b)

in denen X, Y, $R_1$, $R_2$, $R_3$ und $R_4$ die oben angegebenen Bedeutungen besitzen, $R_c$ ein Wasserstoffatom bedeutet und $R_a$ ein Wasserstoffatom bedeutet in dem Fall, da das verwendete Reagens Ammoniak ist, oder eine Gruppe ausgewählt aus geradkettigem oder verzweigtem ($C_1$-$C_6$)-Alkyl, Aryl und geradkettigem oder verzweigtem Aryl-($C_1$-$C_6$)-alkyl in dem Fall, da das verwendete Reagens ein primäres Amin ist,
von welchen Verbindungen der Formeln (XVIII/a) und (XVIIIb) man die Alkoholfunktion mit einer Schutzgruppe für die Hydroxygruppen schützt und dann der Einwirkung einer Verbindung der Formel (XVI) Hal-$G_1$, wie sie oben definiert worden ist, unterwirft zur Bildung der Verbindungen der Formel (XIX/a) bzw. (XIX/b):

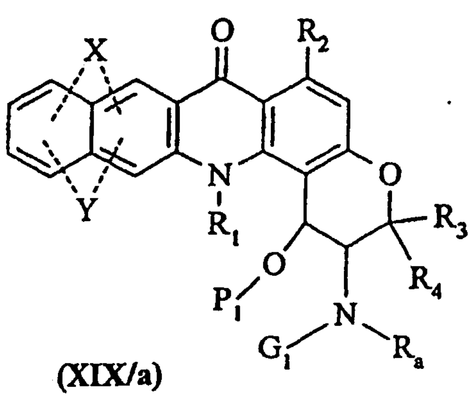

(XIX/a)

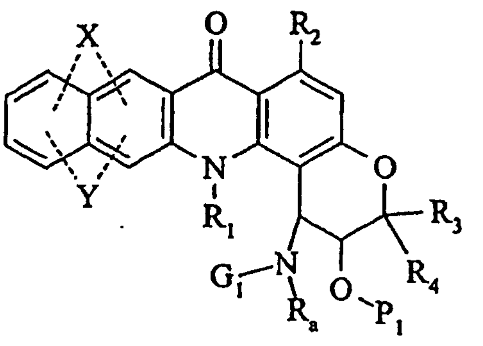

(XIX/b)

in denen X, Y, $R_1$, $R_2$, $R_3$, $R_4$, $R_a$ und $G_1$ die oben angegebenen Bedeutungen besitzen und $P_1$ eine Schutzgruppe für die Hydroxyfunktionen darstellt,
von welchen Verbindungen der Formeln (XIX/a) und (XIX/b) man die Schutzgruppe der Hydroxygruppe abspaltet zur Bildung der Verbindungen der Formeln (I/e1) bzw. (I/f1), Sonderfällen der Verbindungen der Formel (I):

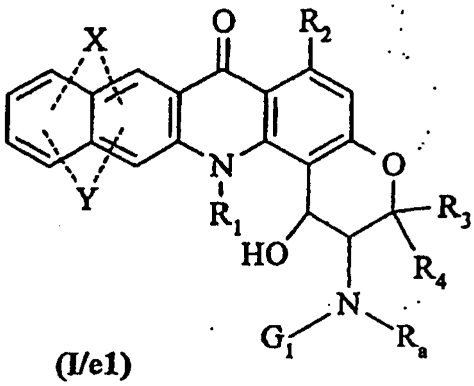

(I/e1)

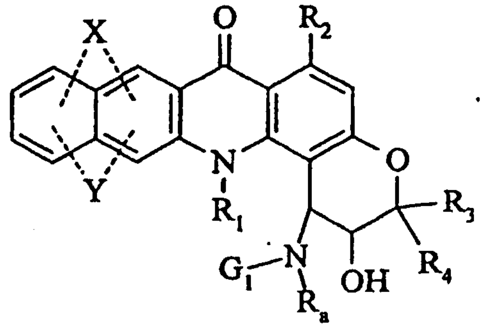

(I/f1)

in denen X, Y, $R_1$, $R_2$, $R_3$, $R_4$. $R_a$ und $G_1$ die oben angegebenen Bedeutungen besitzen, welche Verbindungen der Formeln (I/e1) und (I/f1) man:

❖ entweder der Einwirkung eines Anhydrids der Formel $(R_{30}CO)_2O$, in der $R_{30}$ eine geradkettige oder verzweigte $(C_1\text{-}C_6)$-Alkylgruppe, Arylgruppe oder geradkettige oder verzweigte Aryl-$(C_1\text{-}C_6)$-alkylgruppe bedeutet, unterwirft zur Bildung der Verbindungen der Formeln (I/e2) bzw. (I/f2). Sonderfällen der Verbindungen der Formel (I):

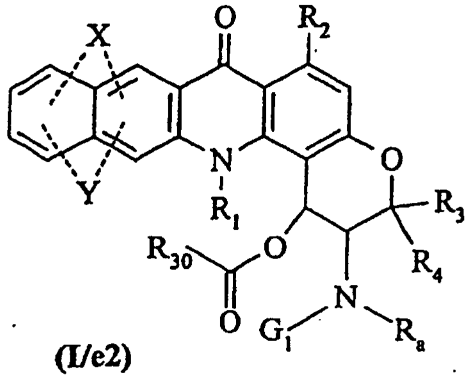

(I/e2)

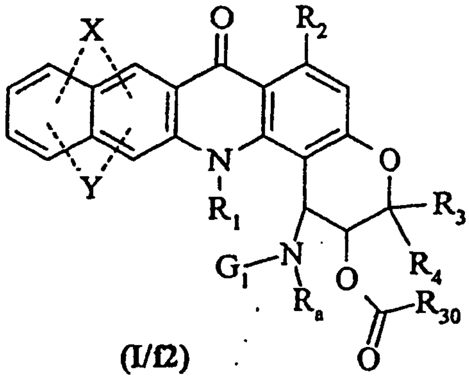

(I/f2)

in denen X, Y, $R_1$, $R_2$, $R_3$, $R_4$, $R_a$, $G_1$ und $R_{30}$ die oben angegebenen Bedeutungen besitzen,
❖ oder Bedingungen der Deshydratation in saurem Medium unterwirft zur Bildung der Verbindungen der Formel (I/e3) bzw. (I/f3), Sonderfällen der Verbindungen der Formel (I):

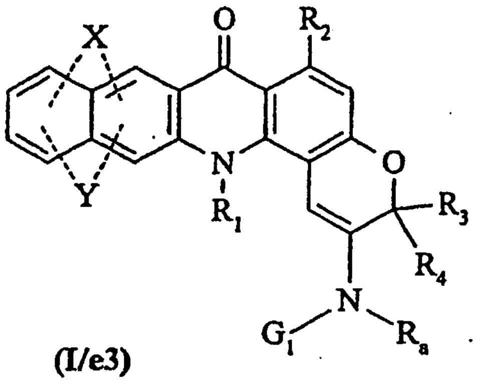

(I/e3)

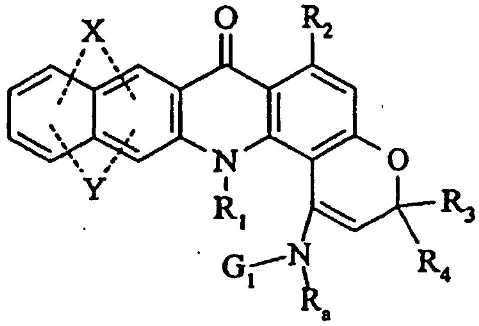

(I/f3)

in denen X, Y, $R_1$, $R_2$, $R_3$, $R_4$, $R_a$ und $G_1$ die oben angegebenen Bedeutungen besitzen,

c) ⇒ oder der Einwirkung von $NaN_3$ in Gegenwart von Wasserstoffperoxid gefolgt von einer Reduktionsstufe unterwirft,
zur Bildung der Verbindungen der Formel (XX):

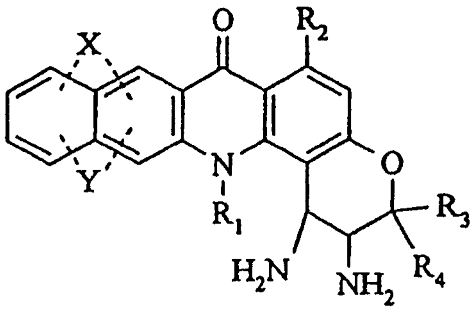

**(XX)**

in der X, Y, $R_1$, $R_2$, $R_3$ und $R_4$ die oben angegebenen Bedeutungen besitzen,
welche Verbindungen der Formel (XX) der Einwirkung einer Verbindung der Formel (XVI) Hal-$G_1$, wie sie oben definiert worden ist, unterworfen werden zur Bildung der Verbindungen der Formel (I/g), einem Sonderfall der Verbindungen der Formel (I):

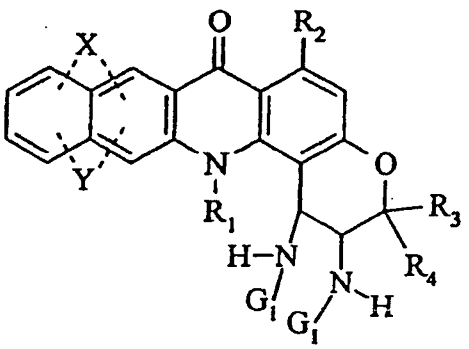

**(I/g)**

in der X, Y, $R_1$, $R_2$, $R_3$, $R_4$ und $G_1$ die oben angegebenen Bedeutungen besitzen,
d) ⇒ oder der Einwirkung von Kaliumpermanganat in polarem Medium unterwirft zur Bildung der Verbindungen der Formel (XXI):

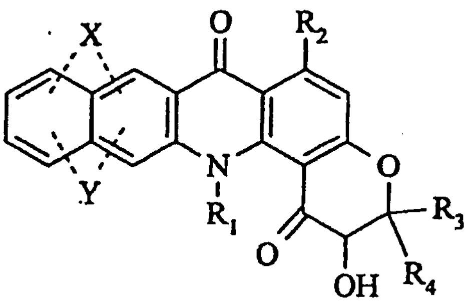

**(XXI)**

in der X, Y, $R_1$, $R_2$, $R_3$ und $R_4$ die oben angegebenen Bedeutungen besitzen,
von welchen Verbindungen der Formel (XXI) man die Hydroxyfunktion durch eine in klassischer Weise in der organischen Synthese verwendete Schutzgruppe schützt und dann der Einwirkung von $P_2S_5$ unterwirft zur Bildung der Verbindungen der Formel (XXII):

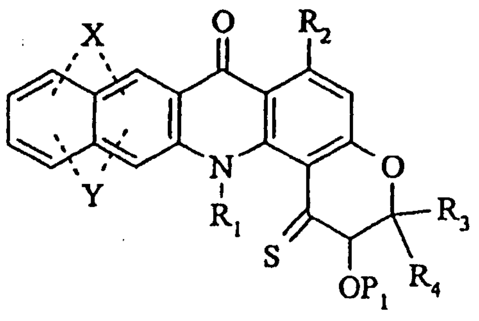

(XXII)

in der X, Y, $R_1$, $R_2$, $R_3$ und $R_4$ die oben angegebenen Bedeutungen besitzen und $P_1$ eine Schutzgruppe für die Hydroxyfunktionen darstellt,
welche Verbindungen der Formel (XXII) man mit einem Reduktionsmittel behandelt und dann einer Reaktion zur Abspaltung der Schutzgruppe der Hydroxyfunktion unterwirft zur Bildung der Verbindungen der Formel (XXIII):

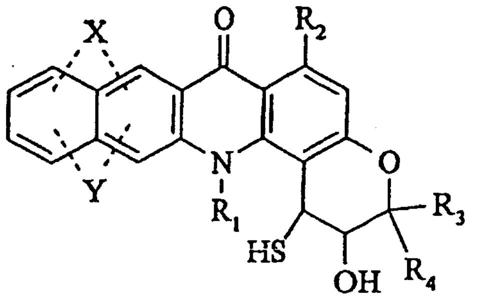

(XXIII)

in der X, Y, $R_1$, $R_2$, $R_3$ und $R_4$ die oben angegebenen Bedeutungen besitzen, welche Verbindungen der Formel (XXIII) man mit einer Verbindung der Formel (XVI), wie sie oben definiert worden ist, umsetzt, zur Bildung der Verbindungen der Formel (I/i), einem Sonderfall der Verbindungen der Formel (I):

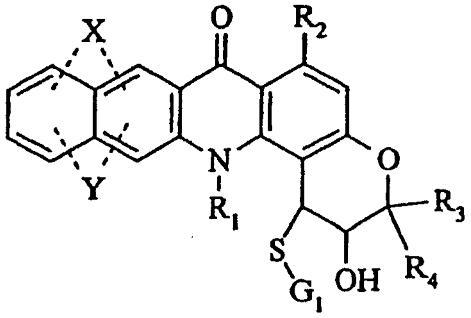

(I/i)

in der X, Y, $G_1$, $R_1$, $R_2$, $R_3$ und $R_4$ die oben angegebenen Bedeutungen besitzen, welche Verbindungen der Formel (I/i) der Einwirkung eines Alkylierungsmittels, eines Acylierungsmittels oder einer Verbindung der Formel (XVIa):

**Hal - G'$_1$** **(XVIa)**

in der Hal ein Halogenatom darstellt und $G'_1$ eine Gruppe bedeutet ausgewählt aus $-C(W_2)-W_3-T_1$, $-C(W_2)-T_1$, $-S(O)_n-W_3-T_1$ und $-S(O)_n-T_1$, in denen $W_2$, $W_3$, $T_1$ und n die bezüglich der Formel (I) angegebenen Bedeutungen besitzen, unterworfen werden können,
zur Bildung der Verbindungen der Formel (I/j), einem Sonderfall der Verbindungen der Formel (I):

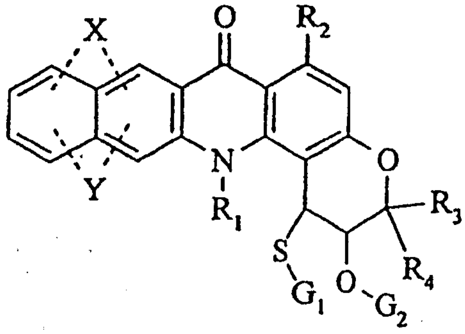

(I/j)

in der X, Y, $G_1$, $R_1$, $R_2$, $R_3$ und $R_4$ die oben angegebenen Bedeutungen besitzen und $G_2$ eine Gruppe bedeutet ausgewählt aus $G'_1$, wie sie oben definiert worden ist, geradkettigem oder verzweigtem ($C_1$-$C_6$)-Alkoxy, geradkettigem oder verzweigtem ($C_1$-$C_6$)-Alkylcarbonyloxy, Arylcarbonyloxy und geradkettigem oder verzweigtem Aryl-($C_1$-$C_6$)-alkylcarbonyloxy,

wobei die Verbindungen (I/a) bis (I/j), (I/$c_1$) bis (I/$c_3$), (I/$d_1$ bis (I/$d_3$), (I/$e_1$) bis (I/$e_3$) und (I/$f_1$) bis (I/$f_3$) die Gesamtheit der erfindungsgemäßen Verbindungen bilden, die man gegebenenfalls mit Hilfe einer klassischen Reinigungsmethode reinigt, gewünschtenfalls mit Hilfe einer klassischen Trennmethode in ihre verschiedenen Isomeren auftrennt, gewünschtenfalls in ihre N-Oxide überführt und gegebenenfalls in ihre Additionssalze mit einer pharmazeutisch annehmbaren Säure oder Base umwandelt.

**9.** Pharmazeutische Zubereitungen enthaltend als Wirkstoff mindestens eine Verbindung nach einem der Ansprüche 1 bis 7 allein oder in Kombination mit einem oder mehreren inerten, nichttoxischen, pharmazeutisch annehmbaren Trägermaterialien oder Hilfsstoffen.

**10.** Pharmazeutische Zubereitungen nach Anspruch 9, enthaltend als Wirkstoff mindestens eine Verbindung nach einem der Ansprüche 1 bis 7, nützlich als Arzneimittel zur Behandlung von Krebs.

**11.** Pharmazeutische Zubereitungen enthaltend als Wirkstoff mindestens eine Verbindung der Formel (XXI), wie sie in Anspruch 8 definiert ist, allein oder in Kombination mit einem oder mehreren inerten, nichttoxischen, pharmazeutisch annehmbaren Trägermaterialien oder Hilfsstoffen.

**12.** Pharmazeutische Zubereitung nach Anspruch 11, enthaltend als Wirkstoff 2-Hydroxy-6-methoxy-3,3-dimethyl-2,3-dihydro-1*H*-benzo[*b*]pyrano[3,2-*h*]acridin-1,7(14*H*)-dion allein oder in Kombination mit einem oder mehreren inerten, nichttoxischen, pharmazeutisch annehmbaren Trägermaterialien oder Hilfsstoffen.

**13.** Pharmazeutische Zubereitung nach einem der Ansprüche 11 oder 12, nützlich als Arzneimittel für die Behandlung von Krebs.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1042326 A


### Littérature non-brevet citée dans la description


- *J. Pharm.. Sci,* 1966, vol. 55 (8), 758-768
- *J. Med. Chem.,* 1996, vol. 39, 4762-4766
- **J. CARMICHAEL et al.** *Cancer Res,* 1987, vol. 47, 936-942